# EUROPEAN PATENT SPECIFICATION

(11) **EP 4 251 200 B1**
(45) Date of publication and mention of the grant of the patent: **30.10.2024**
(21) Application number: 21815535.6
(22) Date of filing: 30.11.2021
(51) Int. Cl.: A61K 39/145, C07K 14/11

(54) **NOVEL REPLICATION DEFICIENT INFLUENZA A VIRUS INDUCING HIGH LEVELS OF TYPE I INTERFERON**
NEUES REPLIKATIONSDEFIZIENTES INFLUENZA-A-VIRUS ZUR INDUKTION HOHER KONZENTRATIONEN VON INTERFERON VOM TYP I
NOUVEAU VIRUS DE LA GRIPPE A DÉFICIENT EN RÉPLICATION INDUISANT DES NIVEAUX ÉLEVÉS D'INTERFÉRON DE TYPE I

(30) Priority: 30.11.2020 EP 20210630
(43) Date of publication of application: 04.10.2023
(73) Proprietor: BlueSky Immunotherapies GmbH, 1060 Vienna (AT)
(72) Inventor: WOLSCHEK, Markus, 1090 Vienna (AT); FERKO, Boris, 1150 Vienna (AT)
(74) Representative: Loidl, Manuela Bettina
(86) International application number: PCT/EP2021/083534
(87) International publication number: WO 2022/112595

(56) References cited:
- WO-A1-2015/063085
- WO-A1-99/64068

## Description

### FIELD OF THE INVENTION

The present invention refers to a novel replication deficient influenza A virus which is inducing high levels of type I interferon (IFN) in cells infected by said virus, comprising an NS1 protein with a functional effector domain and a non-functional RNA binding domain with a deletion of at least 15 amino acids within the N-terminal 80 amino acids of the NS1 protein, wherein the virus comprises the sequence of any of SEQ ID NOs: 3, 5, 7, 9 and 11, and its use for antiviral treatment.

### BACKGROUND OF THE INVENTION

When Influenza A virus enters a cell due to entry into the host cell through endocytosis, stimulus-specific signals are transduced along the interferon signaling pathway to activate antiviral responses. Pattern recognition receptors (PRRs) sense incoming viruses and activate transcription of interferon (IFN) genes, IFNs launch the expression of IFN-stimulated genes (ISGs) in infected cells as well as in nearby non-infected cells, protecting them from potential viral invasion. The ISGs encode a variety of antiviral proteins with diverse modes of action, regulation of apoptosis; production of neuro- and immuno-modulators; cytokines and chemokines for activation and recruitment of immune cells to the site of infection, GTP catabolism and cytokine processing; STAT1 for amplification of autocrine ISG expression, as well as many other antiviral factors. As a result, ISG products can inhibit viral replication in infected cells, alert non-infected cells for potential infections, attract immune cells, and trigger an alarm in the central nervous system about the ongoing infection (Shim J.M. et al., Viruses, 2017, 9(8), 223).

The emergence of newly identified viruses highlights the need for the development of novel antiviral strategies.

Experimental and preliminary clinical studies showed that treatment with type I IFNs was beneficial *in vitro,* in experiments with animals as well as in patients with SARS (Loutfy et al., 2003; Cinatl et al., 2003, Haagmans et al., 2004, Hewnsley et al., 2004).

Severe acute respiratory syndrome coronavirus 2 (SARS-CoV-2) is a newly emerged coronavirus which causes a severe acute respiratory disease, COVID-19. COVID-19 first appeared in Wuhan, a city in China, in December 2019. SARS-CoV is an animal virus, perhaps bats are the reservoir of the virus, which spread to other animals including humans. The transmission of SARS-CoV is primarily from human to human. These viruses generally target the respiratory system of a patient and lead to influenza-like symptoms. Coronaviruses are enveloped spherical particles, the spike glycoproteins (S protein) of which form a crown-like surface. The S protein consists of two subunits: S1 and S2. The fragment located in the middle of the S1 subunit (amino acids (aa) 318-510 with respect to the S1 subunit sequence) is the minimum receptor-binding domain (RBD) in SARS-CoV, which binds to the host cell receptor angiotensin converting enzyme 2 (ACE2). The RBD is about 192 amino acids long and comprises the receptor binding motif (RBM). The binding of RBD and ACE2 triggers the conformational change of the S2 subunit and virus particle invasion.

As of November 28, 2020, the World Health Organization has reported about 62 million confirmed cases world-wide, resulting in 1,45 million deaths.

Symptoms of COVID-19 can range from mild-illness to pneumonia, renal dysfunction, respiratory and multi-organ failure. Unlike the previous SARS-CoV, COVID-19 has proved to be more lethal. Patients infected with COVID-19 rely on their natural immunity and generally seek supportive care to help relieve symptoms. In severe cases, treatment involves mechanical ventilation and vital organ function support.

There are few clinical experiences with the IFN treatment of coronavirus infections. In an experimental setting, 55 healthy volunteers were treated with intranasal recombinant IFN or placebo for 15 days and were exposed to coronavirus by direct intranasal inoculation on the eighth day of treatment (Turner et al., 1986). The therapy with IFN shortened the duration and reduced the severity of coronavirus cold symptoms, suggesting that intranasal recombinant IFN-α may be an effective prophylactic treatment for coronavirus infection in humans. Similarly, intranasal sprays of IFNs given 1 day before and for 3 days after virus challenge protected human volunteers from infection with coronavirus (Tyrell, 1986). Two clinical reports described the use of IFN for treating SARS patients (Zhao et al., 2003; Loutfy et al., 2003). The first study related to the treatment of 190 SARS-patients from Guangzhou, the capital of Guangdong (Zhao et al., 2003). The authors concluded that the best outcome was achieved by the combination of high-dose steroids with quinolone plus azithromycin. No significance for IFN-α was observed in this study. The second study used a modified IFN-α, IFN alfacon-1 (Infergen^{®}, Intermune, Brisbane, California, USA). IFN alfacon-1 is a non-naturally occurring synthetic recombinant type I IFN-α that contains the most common amino acids from 13 IFN-α non-allelic subtypes in each amino acid position. Its specific activity against numerous viruses was higher than that exhibited by other IFN-α agents, indicating its higher antiviral activity on a molar basis (Melian and Plosker, 2001). In the preliminary, uncontrolled study of patients with SARS, 13 patients who received single treatment with corticosteroids were compared with 9 patients who additionally received IFN alfacon-1 (Loutfy et al., 2003). Amongst other favorable parameters, the use of IFN alfacon-1 resulted in a more rapid resolution of radiographic lung abnormalities and better oxygen saturation levels. Due to these encouraging results, Health Canada has already approved a protocol for a trial with alfacon-1 in case of a re-emergence of SARS.

Experimental and pilot clinical data strongly suggest that type I IFNs are promising candidates for SARS treatment protocols. They may not only be used as inhibitors of SARS-CoV replication but also to improve deregulated immune responses and inflammatory processes that are known to contribute to SARS. Although most studies focus on the evaluation and clinical use of IFN-α, IFN-β was superior to IFN-α in terms of SARS-CoV replication inhibition (Cinatl et al., 2003).

Today, IFN is established as an essential component of hepatitis B and C treatment regimens (Friedman 2008). Even though clinical studies demonstrated that intranasal IFN treatment can also prevent rhinovirus infection and spread (McKinlay, 2001) side effects such as irritation of the nasal mucosa and occasional nose prevented further in-depth evaluation of the potential therapeutic use of IFNs to combat respiratory diseases such as influenza.

Thus far, there has been no therapeutic agent to prevent or successfully treat SARS-CoV-2 infection. In view of the continuing threat to human health, there is an urgent need for preventive and therapeutic antiviral therapies for SARS-CoV-2 control but also for other viral threats.

Thus, there is a need for a composition for the prevention and treatment of virus infections and their associated complications.

### SUMMARY OF THE INVENTION

It is the objective of the present invention to provide a composition for preventing viral infections.

The objective is solved by the subject matter as set out in the appended claims.

Effective innate immune response against viral infection relies heavily on the IFN type I responses and their downstream cascade that culminates in controlling viral replication and induction of effective adaptive immune response. A successful mounting of this type I IFN response is able to suppress viral replication and dissemination at an early stage.

Basically, all viruses employ multiple strategies to interfere with type I IFN production and/or the signaling downstream. In particular, for SARS-CoV-2 this dampening strategy is closely associated with the disease severity. Innate immune response plays a crucial role in protective or destructive responses and may open a window for immune intervention. A strong innate immune response is a critical factor for disease outcome. Effective innate immune response against viral infection relies heavily on the IFN type I responses and their downstream cascade that culminates in controlling viral replication and induction of effective adaptive immune response. A successful mounting of this type I IFN response suppresses viral replication and dissemination at an early stage.

It has been surprisingly shown by the inventors that high levels of type I interferon in cells infected by the virus described herein (deINS^{IFN} virus variant) were induced by said novel replication deficient deINS^{IFN} virus variant which comprises an NS1 protein with a functional effector domain and a non-functional RNA binding domain with a deletion of at least 15 amino acids within the N-terminal 80 amino acids of the NS1 protein.

Specifically, inducing high levels of interferon and other cytokines but being fully replication deficient in interferon competent cells, the inventive virus also showed high replication rates and grows to titers above 8log10 in interferon-deficient cells. By inducing the high levels of interferon, the inventive virus described herein strongly attenuates or fully inhibits infection with and replication of other viruses, specifically with IFN sensitive viruses.

The high levels of IFN and other cytokines induced through infection with influenza virus variants and influenza variant based vectors prevent or strongly attenuate an infection with another virus. Therefore, the inventive influenza A virus described herein has the potential to prevent or limit virus induced diseases in general.

The influenza A viruses and vectors described herein can induce a high level of IFN which triggers and enhances an immediate unspecific immune response (innate immunity) and a longer-term specific immune response (adaptive immunity). It also stimulates the production of antiviral molecules.

Immunostimulation/Immunomodulation:
- activates natural killer cells (NK)
- activates cytotoxic T cells (CTL)
- activates dendritic cells (DC)
- inhibits immunosuppressive regulatory T cells (Treg)

Antiviral molecules/Inhibition of viral replication:
- ISG15 (IFN-stimulated gene 15)
- OAS (oligoadenylate synthetase)
- PKR (protein kinase R)
- Mx (IFN-induced GTP binding protein)

Prophylactic treatment with the influenza virus variants described herein induces an innate antiviral response which provides protection against IFN-sensitive viruses.

Therefore, the influenza A virus described herein is not only a promising tool to combat cancer but could be an entirely new method in fighting infectious diseases.

According an embodiment of the invention, the influenza A virus described herein comprises deletions of the N-terminal amino acids 15 to 73 (deINS15-73, specifically influenza A/New Caledonia like virus strain IVR116 lacking amino acids 15-73 of the NS1 protein), 35 to 50 (deINS35-50, specifically influenza A/New Caledonia like virus strain IVR116 lacking amino acids 35-50 of the NS1 protein), 35 to 70 (deINS35-70, specifically influenza A/New Caledonia like virus strain IVR116 lacking amino acids 35-70 of the NS1 protein), 40 to 60 (deINS40-60, specifically influenza A/New Caledonia like virus IVR116 lacking amino acids 40 to 60 of the NS1 protein), or 40 to 80 (deINS40-80, specifically influenza A/New Caledonia like virus strain IVR116 lacking amino acids 40 to 80 of the NS1 protein) with reference to the numbering of SEQ ID NO:1.

According to a specific embodiment, the influenza A virus comprises the sequence of any one of SEQ ID NOs: 3, 5, 7, 9, and 11.

Herein provided is also a pharmaceutical preparation comprising the influenza A virus described herein, optionally in combination with a pharmaceutically acceptable carrier.

Provided herein is also the influenza A virus or the pharmaceutical preparation described herein, for use in the preparation of a medicament.

Further provided is the use in an antiviral treatment of a subject, specifically in the prophylactic or therapeutic treatment of an infection caused by IFN-sensitive viruses.

According to an embodiment, the influenza A virus variant described herein is for use in the treatment of a subject suffering from or going to suffer from virus infection.

According to an embodiment, the influenza A virus, or the pharmaceutical preparation according described herein is for use in the preparation of a medicament for therapeutic treatment of a subject, specifically for use in the preparation of a medicament for therapeutic treatment of a subject in need of antiviral treatment.

Specifically, the antiviral treatment is effective against IFN-sensitive viruses.

According to a specific embodiment, the IFN-sensitive viruses are selected from the group of coronavirus, influenza virus, respiratory syncytial virus, metapneumovirus, parainfluenza virus, flaviviruses, hepatitis virus, herpes simplex virus, rhinovirus and vaccinia virus.

Specifically, the coronavirus is β-coronavirus, SARS-CoV-2, MERS-CoV, SARS-CoV-1, HCoV-OC43, HCoV-HKU1, and an α-coronavirus, such as HCoV-NL63, HCoV-229E or PEDV.

Specifically, early treatment with the influenza A virus variant (deINS^{IFN} virus variant) described herein has the potential to abolish virus, specifically SARS-CoV, and influenza virus replication. Basically, by inducing IFN-β, deINS^{IFN} variants described herein will permit the innate immune system to overcome the IFN antagonist functions of the candidates. In other words, the blockage of IFN through the respective IFN antagonists will be compensated by IFN induction, specifically type I interferon, specifically β interferon induction, through deINS^{IFN} virus variants.

Specifically, it is considered advantageous to induce IFN locally at the site of viral entry than providing it exogenously (topically) or systemically, thus omitting the side effects of a conventional local or systemic application of interferon.

According to an embodiment, the influenza virus or the pharmaceutical preparation described herein is for use in the treatment of a disease condition caused by an IFN-sensitive virus, wherein said disease condition is influenza, common cold, infection of the nose, sinusitis, throat and larynx, bronchiolitis, diarrhea, rash on skin, or pneumonia, acute respiratory distress syndrome (ARDS).

Specifically, the pharmaceutical preparation described herein is formulated for local administration, preferably for application to the upper and lower respiratory tract, nasal, pulmonary, intraoral, ocular, or dermal use, or for systemic administration, preferably by intravenous, intramuscular, subcutaneous, intradermal, transdermal, or oral administration.

Specifically, it is formulated for nasal administration. IFN induction by intranasal application of the influenza A virus variant described herein is safe and well tolerated.

Specifically, the preparation is administered to the subject as a spray, a powder, a gel, an ointment, a cream, a foam, or a liquid solution, a lotion, a gargle solution, an aerosolized powder, an aerosolized liquid formulation, granules, capsules, drops, tablet, syrup, lozenge, or a preparation for infusion or injection.

According to a specific embodiment, the pharmaceutical preparation is administered as the sole antiviral substance, or wherein treatment is combined with a further treatment with one or more active substances, preferably selected from the group consisting of antiviral, and antibiotic substances.

In an embodiment a subject is treated which has been infected or is at risk of being infected with an IFN-sensitive virus, preferably it is a human being, dog, cat, horse, camelid, cattle or pig.

In an embodiment herein provided is the influenza A virus, or the pharmaceutical preparation described herein for use in inhibiting replication of IFN sensitive viruses.

Also provided herein but not claimed is a method for increasing interferon production in a subject with the influenza A virus of the invention, comprising the steps of administering an effective amount of the influenza A virus to the subject.

Specifically, influenza A virus is administered to the subject on a plurality of occasions.

Specifically, the influenza A virus according to the invention further comprises modifications of the NA and HA proteins.

In a further embodiment, the influenza A virus comprises modifications of the PB1, PB2, PA and or NP proteins.

Further provided herein is an isolated nuclei acid sequence expressing the replication deficient influenza A virus described herein.

Specifically, the isolated nucleic acid sequence comprises any one of SEQ ID NOs: 4, 6, 8, 10, and 12.

### FIGURES

Figure 1: Growth of NS1 deletion mutants in interferon-competent cells. Human fibroblasts were infected with influenza wild-type virus (WT NS) and different deletion mutants: deINS1 has a complete NS1 deletion, for the other deletion mutants the deleted amino acids of the NS1 protein are indicated.
Figure 2: Growth of NS1 deletion mutants in interferon-deficient Vero cells. Cells were infected with different deletion mutants and viral titers were determined. The length of the NS1 deletion is indicated, deINS1 refers to a full deletion of the NS1 protein.
Figure 3: Interferon-beta induction by NS1 deletion mutants. Human fibroblasts were infected with wild-type influenza virus (WT NS) and different deletion mutants. deINS1 has a complete NS1 deletion, for the other deletion mutants the deleted amino acids of the NS1 protein are indicated.

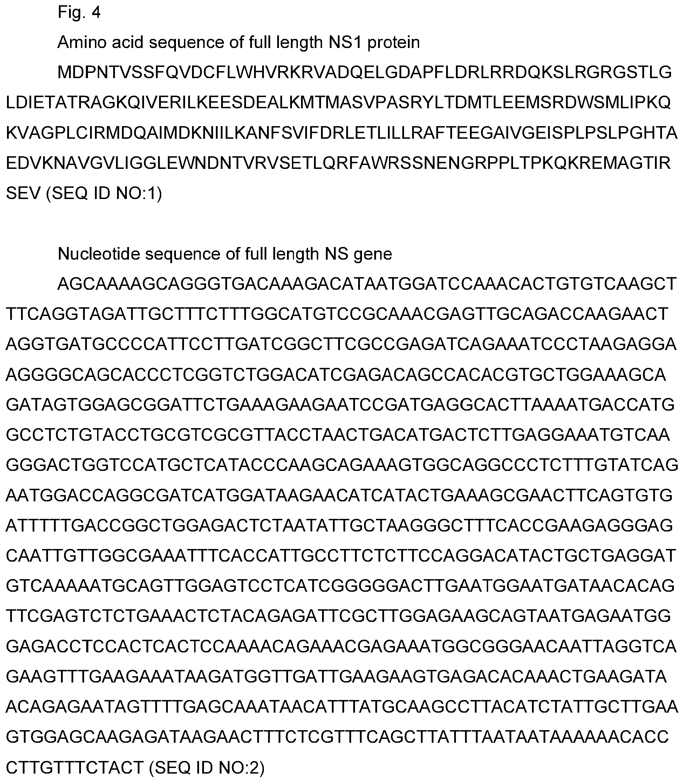

Figure 4: Nucleotide and amino acid sequence of PR8 NS1 protein.
Figure 5: Ferrets were treated with influenza deINS40-60 before (12h) and after (12h and 24h) challenge infection with influenza A H1N1 pdm09 like wild type virus. The lung titers of the challenge virus were determined at day 2 after infection. The difference in titers between treated and untreated animals was significant (p=0,001).
Figure 6: K18-hACE2 mice were treated with influenza deINS40-60 before (1d) challenge infection with Sars-CoV2 (UVE/SARS-CoV-2/2020/FR/702). The lung titers of the challenge virus were determined at day 3 after infection. The difference in titers between treated and untreated animals was highly significant (p<0,0001).
Figure 7: K18-hACE2 mice were treated with influenza deINS40-60 before (1d) challenge infection with Sars-CoV2 (UVE/SARS-CoV-2/2020/FR/702) and weight loss was monitored. In the figure weight loss at day 5 after challenge is shown. The difference in weight loss between treated and untreated animals was highly significant (p= 0,0003).

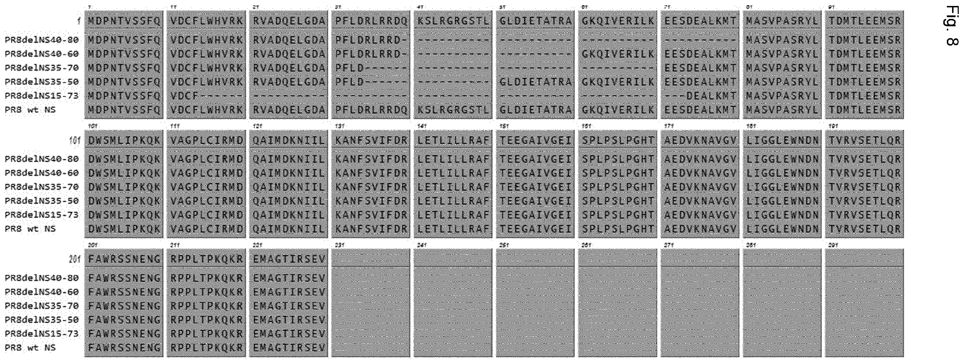

Figure 8: Amino acid sequence alignment of deINS40-80 (SEQ ID NO:11), deINS40-60 (SEQ ID NO:9), deINS35-70 (SEQ ID NO:7), deINS15-73 (SEQ ID NO:3), wild type NS1 (SEQ ID NO:1) in PR8 virus backbone.

### DETAILED DESCRIPTION

Unless indicated or defined otherwise, all terms used herein have their usual meaning in the art, which will be clear to the skilled person. Reference is for example made to the standard handbooks, such as Sambrook et al, "Molecular Cloning: A Laboratory Manual" (4th Ed.), Vols. 1 -3, Cold Spring Harbor Laboratory Press (2012); Krebs et al., "Lewin's Genes XI", Jones & Bartlett Learning, (2017), and Murphy & Weaver, "Janeway's Immunobiology" (9th Ed., or more recent editions), Taylor & Francis Inc, 2017.

The subject matter of the claims specifically refers to artificial products or methods employing or producing such artificial products, which may be variants of native (wild-type) products. Though there can be a certain degree of sequence identity to the native structure, it is well understood that the materials, methods and uses of the invention, e.g., specifically referring to isolated nucleic acid sequences, amino acid sequences, fusion constructs, expression constructs, transformed host cells and modified proteins, are "man-made" or synthetic, and are therefore not considered as a result of "laws of nature".

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

The term "about" as used herein refers to the same value or a value differing by +/-5 % of the given value.

As used herein and in the claims, the singular form, for example "a", "an" and "the" includes the plural, unless the context clearly dictates otherwise.

As used herein, amino acids refer to twenty naturally occurring amino acids encoded by sixty-one triplet codons. These 20 amino acids can be split into those that have neutral charges, positive charges, and negative charges:
The "neutral" amino acids are shown below along with their respective three-letter and single-letter code and polarity: Alanine(Ala, A; nonpolar, neutral), Asparagine (Asn, N; polar, neutral), Cysteine (Cys, C; nonpolar, neutral), Glutamine (Gln, Q; polar, neutral), Glycine (Gly, G; nonpolar, neutral), Isoleucine (Ile, I; nonpolar, neutral), Leucine (Leu, L; nonpolar, neutral), Methionine (Met, M; nonpolar, neutral), Phenylalanine (Phe, F; nonpolar, neutral), Proline (Pro, P; nonpolar, neutral), Serine (Ser, S; polar, neutral), Threonine (Thr, T; polar, neutral), Tryptophan (Trp, W; nonpolar, neutral), Tyrosine (Tyr, Y; polar, neutral), Valine (Val, V; nonpolar, neutral), and Histidine (His, H; polar, positive (10%) neutral (90%)).

The "positively" charged amino acids are: Arginine (Arg, R; polar, positive), and Lysine (Lys, K; polar, positive).

The "negatively" charged amino acids are: Aspartic acid (Asp, D; polar, negative), and Glutamic acid (Glu, E; polar, negative).

There are three general types of influenza viruses, Type A, Type B and Type C, which are defined by the absence of serological cross-reactivity between their internal proteins. Influenza Type A viruses are further classified into subtypes based on antigenic and genetic differences of their glycoproteins, the HA and NA proteins. Most of all the known HA and NA subtypes (H1 to H17 and N1 to N10) have been isolated from birds, which are thought to act as a natural reservoir for influenza.

The **influenza virion** consists of an internal ribonucleoprotein core (a helical nucleocapsid) containing the single-stranded RNA genome, and an outer lipoprotein envelope lined inside by a matrix protein (M1). The segmented genome of influenza A and B virus consists of eight segments, seven for influenza C, of linear, negative polarity, single-stranded RNAs which encode eleven, some influenza A strains ten, polypeptides, including the RNA-dependent RNA polymerase proteins (PB2, PB1 and PA) and nucleoprotein (NP) which form the nucleocapsid; the matrix membrane proteins (M1, M2 or BM2 for influenza B, respectively); two surface glycoproteins which project from the lipid containing envelope: hemagglutinin (HA) and neuraminidase (NA); the nonstructural protein (NS1) and the nuclear export protein (NEP, also: NS2). Influenza B viruses encode also NB, a membrane protein which might have ion channel activity and most influenza A strains also encode an eleventh protein (PB1-F2) believed to have proapoptotic properties. Transcription and replication of the genome takes place in the nucleus and assembly occurs via budding on the plasma membrane. The viruses can reassort genes during mixed infections. Influenza virus adsorbs via HA to sialyloligosaccharides in cell membrane glycoproteins and glycolipids. Following endocytosis of the virion, a conformational change in the HA molecule occurs within the cellular endosome which facilitates membrane fusion, thus triggering uncoating. The nucleocapsid migrates to the nucleus where viral mRNA is transcribed. Viral mRNA is transcribed and processed by a unique mechanism in which viral endonuclease cleaves the capped 5'-terminus from cellular heterologous mRNAs which then serve as primers for transcription from viral RNA templates by the viral transcriptase. Transcripts terminate at sites 15 to 22 bases from the ends of their templates, where oligo(U) sequences act as signals for the addition of poly(A) tracts. Of the eight viral RNA molecules of influenza A virus so produced, six are monocistronic messages that are translated directly into the proteins representing HA, NA, NP and the viral polymerase proteins, PB2, PB1 and PA. The other two transcripts undergo splicing, each yielding two mRNAs which are translated in different reading frames to produce M1, M2, NS1 and NS2. In most of influenza A viruses, segment 2 also encodes for a second protein (PB1-F2), expressed from an overlapping reading frame. In other words, the eight viral RNA segments code for eleven proteins: nine structural and 2 non-structural (NS1, PB1-F2) proteins.

A **"recombinant"** virus is one which has been manipulated in vitro, e.g., using recombinant DNA techniques, to introduce changes to the viral genome, or otherwise artificially generated.

Influenza virus wherein the NS1 protein is fully deleted (deINS1 virus) stimulates increased levels of type I IFNs, due to lack of its IFN-antagonist NS1. Surprisingly it had been shown that targeted deletion of at least 15 amino acids within the N-terminal 80 amino acids of the NS1 protein a significant increase of the level of IFN induction compared to deINS1 virus was achieved while growth properties of the virus in Interferon deficient cells was also improved compared to deINS1 virus, whereas replication in normal cells was prevented. Therefore, these virus variants provide excellent antiviral properties.

The truncated **NS1 protein** of the herein described recombinant influenza virus has a deletion of at least 15 amino acids within the N-terminal 80 amino acids of the NS1 protein and thus the virus lacks a functional NS1 protein but preserves its effector domain functionality. It may be referred to it as NS1 influenza virus variant. Specifically, said variants comprise deletions of the N-terminal amino acids 14 to 73, or 35 to 50, or 35 to 70, or 40 to 60, or 40 to 80 with reference to the numbering of wt PR8 NS1 protein (SEQ ID NO:1), wherein the amino terminal acid is number 1.

Said targeted partial deletions of the functional NS1 protein lead to a significant attenuation of influenza virus due to lack of replication in interferon competent cells or organisms (replication deficient phenotype). Viruses comprising NS1 deletions as described herein are not able to antagonize cytokine production of infected cells, therefore inducing self-adjuvanting and immune modulating effects. The hallmark of immune response after immunization with the inventive recombinant influenza virus is increase of type I interferon production in infected cells.

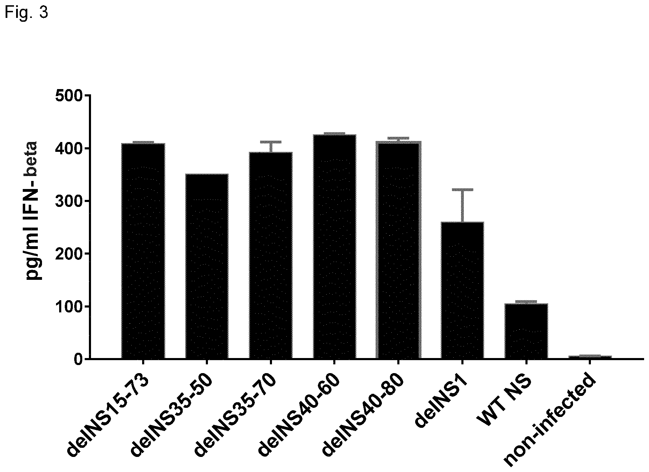

The lack of NS1 activity achieves highly advantageous properties for the replication deficient virus. Specifically, when delivered intranasally, it infects cells of the upper respiratory tract and expresses viral antigens, but it does not form viral progeny and the vaccine strains are not shed by the recipient, making replication deficient deINS1^{IFN} virus variant very safe; and, additionally, since these NS1 depleted strains are unable to counteract the host interferon (IFN) response, infection induces high levels of interferon (> 300pg/ml as determined by ELISA, Fig.3) achieving a natural adjuvant effect that activates also B and T cell-mediated immune responses.

Type I interferons are a large subgroup of interferon proteins that help regulate the activity of the immune system. Interferons bind to interferon receptors. All type I IFNs bind to a specific cell surface receptor complex known as the IFN-α receptor (IFNAR). The mammalian types are designated IFN-α (alpha), IFN-β (beta), IFN-κ (kappa), IFN-δ (delta), IFN-ε (epsilon), IFN-τ (tau), IFN-ω (omega), and IFN-ζ (zeta, also known as limitin). Using the replication deficient influenza A virus variant described herein, the infected cells specifically produce increased levels of interferon-beta compared to uninfected cells.

According to the invention, the term **"replication deficient"** is defined as replication rate in interferon competent host cells that is at least reduced by 95%, preferably 99%, preferably 99,9% compared to wild type influenza virus replication rate, determined by hemagglutination assay, TCID50 assay or plaque assay as well known in the art. Specifically, the influenza virus is completely replication deficient.

The influenza virus described herein can be human or animal influenza virus, such as, but not limited to avian, equine, and swine. Preferably, it is human influenza virus.

The replication deficient influenza A virus comprises an NS1 protein with a functional effector domain, which is located at the C-terminus of the NS1 protein (specifically comprising amino acids 79-230) and a non-functional RNA binding domain located at the N-terminus of the NS1-protein with a deletion of at least 15 amino acids within the N-terminal 80 amino acids of the NS1 protein, specifically comprising deletions of the N-terminal amino acids 14 to 73, 35 to 50, 35 to 70, 40 to 60, or 40 to 80 with reference to wild type NS1 protein having an NS1 protein of SEQ ID NO:1. Specifically, the NS1 protein comprises any one of SEQ ID NOs:3, 5, 7, 9, and 11.

As an alternative embodiment, the influenza virus variant comprises a deletion of 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 48, 49, 60, 61, 62, 63, 64, 65, 66, 67, 68, 69, 70, 71, 72, 73, 74, 75, 76, 77, 78, 79, 80 within the N-terminal 80 amino acids of the NS1 protein.

The term "functional effector domain" refers to an NS1 effector domain that is able to trigger the suppression of host transcription and to mediate global deregulation of host transcription termination. The effector domain specifically inhibits reporter gene expression and interacts with CPSF30 (CPSF4, cleavage and polyadenylation specificity factor 4) or other CPSF subunits.

According to the invention, the recombinant influenza virus comprises modified hemagglutinin (HA) and/or neuraminidase (NA) polypeptides.

On account of their external localization the HA and NA antigens represent the most important viral target structures for the host immune system. Of antibodies which bind specifically to HA, it is thought that they neutralize the viral infectivity, probably by blocking the early steps of infection (Kida et al., 1983). NA-specific antibodies normally do not prevent the initial infection of a target cell but precisely the spread of the virus. In addition, due to competition mechanisms, the immunologic response to NA appears to be partly suppressed in favor of the more frequently occurring HA antigen (Kilbourne, 1976).

The **neuraminidase** (NA) assembles as a tetramer of four identical polypeptides and, when embedded in the envelope of the virus, accounts for approximately 10-20% of the total glycoproteins on the virion surface, with about 40-50 NA spikes and 300-400 HA spikes on an average sized virion of 120nm (McAuley J.L. et al., 2019, Varghese et al., 1983; Ward et al., 1983; Moules et al., 2010). The four monomers, each of approximately 470 amino acids, fold into four distinct structural domains: the cytoplasmic tail, the transmembrane region, the stalk, and the catalytic head. Suggesting that the NA cytoplasmic tail is involved in critical viral functions, the N-terminal domain sequence is nearly 100% conserved across all live attenuated virus subtypes. Reverse engineered viruses containing site-specific mutations in this domain exhibit altered virion morphology and reduced replicative yields (Mitnaul et al., 1996; Jin et al., 1997; Barman et al., 2004). Live attenuated viruses engineered to encode an NA lacking a cytoplasmic tail could still be rescued but with a markedly attenuated phenotype (Garcia-Sastre and Palese, 1995). The altered morphology and attenuated infectivity of viruses expressing NA lacking the cytoplasmic tail domain are thought to be due to a lack of interaction with the membrane-associated matrix M1 viral protein (McAuley J.L. et al., 2019, Enami and Enami, 1996), which ultimately alters the efficiency of budding from the infected host cell (Jin et al., 1997; Ali et al., 2000; Barman et al., 2001; Mintaev et al., 2014). Determinants in both the cytoplasmic tail domain and the transmembrane domain contribute to the transport of the glycoprotein to the apical plasma membrane. However, the role of the tail domain in packaging the surface NA into virions remains unclear. A complete loss of the tail domain (Garcia-Sastre and Palese, 1995) resulted in a 50% reduction in the amount of NA in infected cells. The absence of all tail amino acids except for the initiating methionine gave rise to virus that also showed markedly less incorporation of NA into virions, but in this case, NA was present at the plasma membrane at similar levels to wild-type virus (McAuley J.L. et al., 2019, Mitnaul et al., 1996).

The terms **"hemagglutinin"** and "HA" refer to any influenza virus hemagglutinin. In certain embodiments, the hemagglutinin is influenza hemagglutinin, such as an influenza A hemagglutinin, an influenza B hemagglutinin, or an influenza C hemagglutinin. A typical hemagglutinin comprises domains known to those of skill in the art including a signal peptide (optional), a stem domain (also referred to as a "stalk domain"), a globular head domain, a luminal domain (optional), a transmembrane domain (optional) and a cytoplasmic domain (optional). Functionally, the hemagglutinin glycoprotein is composed of an immunodominant globular head domain involved in virus attachment to the host cell and the membrane proximal stalk/stem domain mediating fusion of the viral and cell membrane in the host endosome. The terms "stalk" and "stem" can be used interchangeably herein. The stalk domain is more conserved among influenza A (group 1 and 2) and B viruses allowing antibodies that target this region to neutralize a wide spectrum of influenza virus subtypes and is identified to harbor neutralizing B-cell epitopes. The immunodominant HA head domains undergo constant antigenic drift or shift The HA stalk domain is composed of three helical bundles and is functionally required for the pH induced conformational changes involved in membrane fusion during viral entry and exit from the host cell. In contrast to the HA-head variability, the stalk domain displays a much higher level of conservation across influenza strains with some central residues being identical across all subtypes (Krystal M, et al., 1982). The stalk domain is evolving at a rate that is significantly slower than that of the head domain. Additionally, the cross-reactive epitopes in the stalk domain targeted by broadly neutralizing monoclonal antibodies are evolving at an even slower rate compared to the full head and stalk regions of the protein (Kirkpatrick E. et al.,2018). Three protective epitopes, with varying levels of cross-reactivity between group 1 and 2 influenza strains, have been identified within the stalk portion of influenza A HA. Epitope 1 is centered on the α-helix of the HA2 region of HA. Targeting this epitope is also protective against B strains, but the antibody must have unique properties to accommodate key modifications helping to obscure the epitope surface (Dreyfus C., et al, 2012). Epitopes 2 and 3 are protective across group 2 influenza A subtypes. Epitope 2 includes the upper portion of the long alpha helix CD in HA2 (Wang T.T. et al., 2010) whereas epitope 3 is located at the base of the HA2 stalk spanning regions of the fusion peptide and helix-capping loops (Ekiert D.C., et al., 2011). The fourth protective stalk epitope is located in the C terminal portion of HA1 and offers broad protection across both B strain lineages (Yasugi M., et al., 2013) Generating a strong antibody response against any of these conserved epitopes can offer broader and more durable protection against influenza by circumventing reliance on epitopes prone to antigenic drift.

Specifically, the influenza virus is a high growth influenza virus specifically comprising one or more amino acid substitutions in the PB1, M and NS2 proteins as described in WO2020152318A.

The term **"antigenically different"** as used herein refers to the presence of different antigenic sites being target by antibody response. Different antigenicity can be due to amino acid substitutions in the HA head domains due to antigenic drifts and shifts of the influenza virus. The 'classical' antigenic sites were historically determined using murine mAbs and analysis of changes in amino acid sequences connected to antigenic drift (as measured by reduction of HI activity). The majority of mutations in the head were focused on sites related to immune escape, while the majority of mutations in the stalk seem to be evenly dispersed throughout the domain. (Kirkpatrick et al., 2018).

As used herein, the term **"infection"** means the invasion by, multiplication and/or presence of a virus, specifically an IFN sensitive virus, in a cell or a subject. An infection can be an "active" infection, i.e., an infection in which the virus is replicating in a cell or a subject. Such an infection is characterized by the spread of the virus to other cells, tissues, and/or organs, from the cells, tissues, and/or organs initially infected by the virus. An infection may also be a latent infection, i.e. an infection in which the virus is not replicating. In certain embodiments, an infection refers to the pathological state resulting from the presence of the virus in a cell or a subject, or by the invasion of a cell or subject by the virus. Herein, infection is infection with an IFN-sensitive virus, such as but not limited to coronavirus, influenza virus, respiratory syncytial virus, metapneumovirus, parainfluenza virus, flaviviruses, hepatitis virus, herpes simplex virus, rhinovirus and vaccinia virus and any combinations thereof.

As used herein, **virus disease** refers to the pathological state resulting from the presence of an IFN sensitive virus in a cell or subject or the invasion of a cell or subject by an IFN sensitive virus. In specific embodiments, the term refers to influenza, common cold, infection of the nose, sinusitis, throat and larynx, bronchiolitis, diarrhea, rash on skin, or pneumonia, acute respiratory distress syndrome (ARDS) caused by said virus.

As used herein, the term **"influenza virus disease"** refers to the pathological state resulting from the presence of an influenza (*e.g*., influenza A or B) virus in a cell or subject or the invasion of a cell or subject by an influenza virus. In specific embodiments, the term refers to a respiratory illness caused by an influenza virus.

As used herein, the term **"corona virus disease"** refers to the pathological state resulting from the presence of a coronavirus (*e.g*., β-coronavirus, such as but not limited to SARS-CoV-2, MERS-CoV, SARS-CoV-1, HCoV-OC43, and HCoV-HKU1) virus in a cell or subject or the invasion of a cell or subject by a coronavirus. In specific embodiments, the term refers to a respiratory illness caused by a coronavirus.

As used herein, the term **"nucleic acid"** includes DNA molecules (e.g., cDNA) and RNA molecules (e.g., mRNA or pre-mRNA) and analogs of the DNA or RNA generated using nucleotide analogs. The nucleic acid can be single-stranded or doublestranded.

As used herein, the terms **"subject"** or **"patient"** are used interchangeably to refer to an animal (*e.g*., birds, reptiles, and mammals). In a specific embodiment, the subject is a mammal including a non-primate (*e.g*., a camel, donkey, zebra, cow, pig, horse, goat, sheep, cat, dog, rat, and mouse) and a primate (*e.g*., a monkey, chimpanzee, and a human). In certain embodiments, the subject is a non-human animal. In some embodiments, the subject is a farm animal or pet. In another embodiment, the subject is a human.

According to a specific embodiment, the pharmaceutical preparation comprising the inventive influenza A virus described herein can further comprise one or more adjuvants.

As used herein, an **"adjuvant"** refers to a substance or mixture that enhances the body's immune response to an antigen, in the present disclosure an adjuvant increases the cellular IFN level.

The term **"stabilizers"** refers to any agent that can increase the stability of the virus, for example it can be bovine serum albumin, sugars, chitosan, dextrans, PEGs etc.

By **"administration"** is meant introducing the pharmaceutical preparation of the present disclosure into a subject; it may also refer to the act of providing the preparation of the present disclosure to a subject (e.g., by prescribing). The preparation can be administered to a human or animal subject in vivo using a variety of known routes and techniques. For example, the preparation may be provided as an injectable solution, suspension or emulsion and administered via parenteral, subcutaneous, oral, epidermal, intradermal, intramuscular, intraarterial, intraperitoneal, intravenous injection using a conventional needle and syringe, or using a liquid jet injection system.

The preparation may be administered topically to skin or mucosal tissue, such as nasally, intratracheally, intestinally, sublingually, rectally or vaginally, or provided as a finely divided spray, such as a mist, suitable for respiratory or pulmonary administration. In certain embodiments, the preparations are administered intramuscularly or intranasally.

The term **"effective amount"** with respect to an antiviral effect as used herein, shall refer to an amount (in particular a predetermined amount) that has a proven antiviral effect. The amount is typically a quantity or activity sufficient to, when applied to a surface or administered to a subject effect beneficial of desired results, including antiviral or clinical results, and, as such, an effective amount or synonym thereof depends upon the context in which it is being applied.

An effective amount of the pharmaceutical preparation is intended to mean that amount of a compound that is sufficient to treat, prevent or inhibit a disease, disease condition or disorder. Such an effective dose specifically refers to that amount of the compound sufficient to result in healing, prevention or amelioration of conditions related to diseases or disorders described herein.

In the context of disease, effective amounts (in particular prophylactically or therapeutically effective amounts) of the influenza A virus as described herein are specifically used to treat, modulate, attenuate, reverse, or affect a disease or condition that benefits from its antiviral effect. The amount of the compound that will correspond to such an effective amount will vary depending on various factors, such as the given drug or compound, the formulation, the route of administration, the type of disease or disorder, the identity of the subject or host being treated, the assessment of the medical situations and other relevant factors, but can nevertheless be routinely determined by one skilled in the art. Specifically, the amount results in an increased production of IFN in a subject treated with the deINS^{IFN} virus variants described herein.

An effective amount can be in the range of between about 1×10⁶ and 1×10¹¹, specifically between about 1×10⁷ to 1×10⁹, specifically about 7×10⁷ to 1×10⁹ TCID50.

A preferred outcome is a significant reduction of fever, weight loss and a reduction of challenge virus in the nasal turbinates and lungs, indicating the antiviral effect. Specifically, the replication deficient influenza A virus is administered to a subject which is going to suffer from a viral infection or to a subject at an early stage of infection.

A treatment or prevention regime of a subject with an effective amount of the deINS^{IFN} virus variant described herein may consist of a single application or administration, or alternatively comprise a series of applications and administrations, respectively. For example, the deINS^{IFN} virus variant described herein may be used at least once a month, or at least once a week, or at least once a day. However, in certain cases of an acute phase, e.g. upon suspected or confirmed exposure to a virus, or after virus infection has been determined, the deINS^{IFN} virus variant described herein may be used more frequently e.g., 1, 2 or-3 times a day.

Specifically, a combination therapy is provided which includes treatment with the preparation described herein and standard therapy of a virus-caused disease, specifically of a coronavirus caused disease.

Doses may be applied in combination with other active agents such as antiviral agents, anti-inflammatory drugs or antibiotics, e.g. upon the subject's risk of viral spread, so to prevent a pathogen associated reaction.

Treatment can be combined with an antiviral, anti-inflammatory or antibiotic treatment, preferably wherein a pharmaceutical preparation is administered before, during (e.g., by co-administration or in parallel), or after said antiviral, anti-inflammatory or antibiotic treatment.

Specifically, the deINS^{IFN} virus variant described herein is combined with an antibiotic such as a beta lactam antibiotic, an aminoglycoside antibiotic, an ansamycin, a carbacephem, a carbapenem, a cephalosporin, a glycopeptide, a lincosamide, a lipopeptide, a macrolide, a monobactam, a nitrofuran, an oxazolidinone, a polypeptide, a sulfonamide, Clofazimine, Dapsone, Capreomycin, Cycloserine, Ethambutol, Ethionamide, Isoniazid, Pyrazinamide, Rifampicin, Rifabutin, Rifapentine, Streptomycin, Arsphenamine, Chloramphenicol, Fosfomycin, Mupirocin, Platensimycin, Quinupristin/Dalfopristin, Thiamphenicol, Tigecycline, Tinidazole, Trimethoprim, Teixobactin, Malacidins, Halicin, clindamycin, vancomycin, metronidazole, fusidic acid, thiopeptides, fidaxomicin, quinolons, tetracyclines, omadacycline, rifamycin, kibdelomycin, oxazolidinone, ketolides, thiazolides, amixicile, teicoplanin, ramoplanin, oritavancin, lantibiotics, capuramycin, surotomycin, thuricin, endolysin, avidocin CD, cadazolid, ramizol, defensins, ridinilazole, medium-chain fatty acids, phages, berberine, lactoferrin.

Specifically, the deINS^{IFN} virus variant described herein is combined with an anti-inflammatory agent such as standard steroidal anti-inflammatory drugs, glucocorticoids and nonsteroidal anti-inflammatory drugs (NSAIDs). Suitable NSAIDs include, but are not limited to ibuprofen, naproxen, fenoprofen, ketoprofen, flurbiprofen, oxaprozin, indomethacin, sulindac, etodolac, ketorolac, diclofenac, nabumetone, piroxicam, meloxicam, tenoxicam, droxicam, lornoxicam, isoxicam, mefenamic acid, meclofenamic acid, mefenamine, flufenamic acid, tolfenamic acid and celecoxib. Suitable steroidal anti-inflammatory agents include, but are not limited to, corticosteroids such as synthetic glucocorticoids.

The pharmaceutical preparation can be formulated as a spray, a powder, a gel, an ointment, a cream, a foam, or a liquid solution, a lotion, a gargle solution, an aerosolized powder, an aerosolized liquid formulation, granules, capsules, drops, tablet, syrup, lozenge, or a preparation for infusion or injection.

The terms **"pharmaceutical preparation"** or "pharmaceutical composition" or "pharmaceutical formulation" refer to the recombinant influenza virus comprising the NS1 protein with a deletion of at least 15 amino acids within the N-terminal 80 amino acids of the NS1 protein as described herein, with other chemical components, such as pharmaceutically acceptable carriers and excipients. One purpose of a pharmaceutical composition is to facilitate administration of a compound to the organism.

As used herein, a **"pharmaceutically acceptable carrier"** refers to a carrier or diluent that does not cause significant irritation to an organism and does not abrogate the biological activity and properties of the administered vaccine compositions. It refers to a diluent, adjuvant, excipient, or vehicle with which the pharmaceutical preparation (e.g., immunogenic or vaccine formulation) is administered. Saline solutions and aqueous dextrose and glycerol solutions can also be employed as liquid carriers, particularly for injectable solutions. Suitable excipients include starch, glucose, lactose, sucrose, gelatine, malt, rice, flour, chalk, silica gel, sodium stearate, glycerol monostearate, talc, sodium chloride, dried skim milk, glycerol, propylene, glycol, water, ethanol and the like. Examples of suitable pharmaceutical carriers are described in "Remington's Pharmaceutical Sciences" by E.W. Martin. The formulation should be selected according to the mode of administration. The particular formulation may also depend on whether the virus is live or inactivated.

Unit-dose or multi-dose containers may be used, for example, sealed ampoules and vials, or single and multi-use sprays, and may be stored comprising a liquid or dry phase, e.g., in a freeze-dried (lyophilized) condition requiring only the addition of the sterile liquid carrier, for example water for injection, immediately prior to use. Preferred unit dosage formulations are those containing a daily dose or unit daily sub-dose, or multiple doses, of the deINS^{IFN} virus variant described herein.

The term "single-dose" as used herein is understood in the following way. A single-dose or amount for single-use is the amount intended for administration that is meant for use in a single subject, such as a patient, either human or animal for a single case/procedure/administration.

The pharmaceutical preparation described herein is specifically provided as human or veterinary medicinal product or pharmaceutical composition.

The influenza virus as described herein can be also useful to prepare reassortant viruses including 6:1:1 reassortants, 6:2 reassortants and 7:1 reassortants. A 6:1:1 reassortant according to the present invention is an influenza virus with 6 internal gene segments, an NA gene segment from a different, second, viral isolate, and a HA gene segment from a third isolate; a 6:2 reassortant is an influenza virus with 6 internal gene segments, and an NA gene segment and a HA gene segment from a different (second) viral isolate; and a 7:1 reassortant is an influenza virus with 6 internal gene segments and an NA gene segment from a vaccine virus, and a HA gene segment from a different viral source than the vaccine virus, or an influenza virus with 6 internal gene segments and a HA gene segment, and an NA gene segment is from a different viral source than the vaccine virus. As an alternative, 5:1:2 reassortants are also encompassed herein.

6:2 reassortant viruses were generated by reverse genetics. NS1 can be derived from any influenza A virus, such as but not limited to A/Puerto Rico/08/1934, A/IVR-116 (a lab strain A virus that contains PB2, PA, NP, M and NS genes from A/Puerto Rico/08/1934 and PB1 from A/Texas/1/1977), A/Hong Kong/4801/2014, A/Texas/1/1977.

Specifically, the NS1 protein is originating from A/Puerto Rico/08/1934.

The replication deficient influenza A virus of the invention can be produced in interferon deficient cells, such as Vero cells by methods well known in the art. Considering a target dose of about 8-9log¹⁰ particles per day and subject, an important prerequisite for technical feasibility is a titer of at least 8log¹⁰ preferably 9log¹⁰ in the upstream process.

In some embodiments, a plurality of vectors incorporating at least the 6 internal genome segments of a one influenza A strain along with one or more genome segments encoding immunogenic influenza surface antigens of a different influenza strain are introduced into a population of host cells. For example, at least the 6 internal genome segments ("the backbone") of a selected influenza A strain, e.g., an artificially engineered influenza A backbone strain encoding the recombinant influenza virus comprising the NS1 segment comprising at least 15 and up to 80 amino acid deletions of the NS1 protein, e.g. but not limited to A/Puerto Rico/08/1934, A/IVR-116, A/Hong Kong/4801/2014, and A/Texas/1/1977 are introduced into a population of host cells along with one or more segments encoding immunogenic antigens derived from another virus strain. Typically, the immunogenic surface antigens include either or both of the hemagglutinin (HA) and/or neuraminidase (NA) antigens. In embodiments where a single segment encoding an immunogenic surface antigen is introduced, the 7 complementary segments of the selected virus are also introduced into the host cells.

In a further embodiment, herein provided is an influenza A virus vector encoding an NS1 protein with a functional effector domain and a non-functional RNA binding domain with a deletion of at least 15 amino acids within the N-terminal 80 amino acids of the NS1 protein. Specifically, said nucleic acid sequences are selected from SEQ ID NOs:4, 6, 8, 10 and 12.

In a further embodiment, herein provided is a plurality of influenza virus vectors for preparing a reassortant influenza A virus comprising a modified NS1 segment, encoding the NS1 protein with a functional effector domain and a non-functional RNA binding domain with a deletion of at least 15 amino acids within the N-terminal 80 amino acids of the NS1 protein described herein, comprising
a) a vector for vRNA production comprising a promoter operably linked to an influenza virus PA DNA linked to a transcription termination sequence, a vector for vRNA production comprising a promoter operably linked to an influenza virus PB1 DNA linked to a transcription termination sequence, a vector for vRNA production comprising a promoter operably linked to an influenza virus PB2 DNA linked to a transcription termination sequence, a vector for vRNA production comprising a promoter operably linked to an influenza virus HA DNA linked to a transcription termination sequence, a vector for vRNA production comprising a promoter operably linked to an influenza virus NP DNA linked to a transcription termination sequence, a vector for vRNA production comprising a promoter operably linked to an influenza virus NA DNA linked to a transcription termination sequence, a vector for vRNA production comprising a promoter operably linked to an influenza virus M DNA linked to a transcription termination sequence, and a vector for vRNA production comprising a promoter operably linked to an influenza virus NS cDNA encoding an NS1 protein with a functional effector domain and a non-functional RNA binding domain with a deletion of at least 15 amino acids within the N-terminal 80 amino acids of the NS1 protein, specifically encoding any of SEQ ID NOs 3, 5, 7, 9, 11, and optionally
b) a vector for mRNA production comprising a promoter operably linked to a DNA segment encoding influenza virus PA, a vector for mRNA production comprising a promoter operably linked to a DNA segment encoding influenza virus PB1, a vector for mRNA production comprising a promoter operably linked to a DNA segment encoding influenza virus PB2, and a vector for mRNA production comprising a promoter operably linked to a DNA segment encoding influenza virus NP, and optionally a vector for mRNA production comprising a promoter operably linked to a DNA segment encoding influenza virus HA, a vector for mRNA production comprising a promoter operably linked to a DNA segment encoding influenza virus NA, a vector for mRNA production comprising a promoter operably linked to a DNA segment encoding influenza virus M1, a vector for mRNA production comprising a promoter operably linked to a DNA segment encoding influenza virus M2, or a vector for mRNA production comprising a promoter operably linked to a DNA segment encoding influenza virus NS2.

In a further embodiment, herein provided is a plurality of influenza virus vectors, comprising
a) a vector for vRNA production comprising a promoter operably linked to an influenza virus PA DNA linked to a transcription termination sequence, a vector for vRNA production comprising a promoter operably linked to an influenza virus PB1 DNA linked to a transcription termination sequence, a vector for vRNA production comprising a promoter operably linked to an influenza virus PB2 DNA linked to a transcription termination sequence, a vector for vRNA production comprising a promoter operably linked to an influenza virus HA DNA linked to a transcription termination sequence, a vector for vRNA production comprising a promoter operably linked to an influenza virus NP DNA linked to a transcription termination sequence, a vector for vRNA production comprising a promoter operably linked to an influenza virus NA DNA linked to a transcription termination sequence, a vector for vRNA production comprising a promoter operably linked to an influenza virus M DNA linked to a transcription termination sequence, and a vector for vRNA production comprising a promoter operably linked to an influenza virus NS cDNA encoding a modified NS segment, encoding an NS1 protein with a functional effector domain and a non-functional RNA binding domain with a deletion of at least 15 amino acids within the N-terminal 80 amino acids of the NS1 protein, specifically encoding any of SEQ ID NOs 3, 5, 7, 9, 11, and
b) a vector for mRNA production comprising a promoter operably linked to a DNA segment encoding influenza virus PA, a vector for mRNA production comprising a promoter operably linked to a DNA segment encoding influenza virus PB1 , a vector for mRNA production comprising a promoter operably linked to a DNA segment encoding influenza virus PB2, and a vector for mRNA production comprising a promoter operably linked to a DNA segment encoding influenza virus NP, and optionally a vector for mRNA production comprising a promoter operably linked to a DNA segment encoding influenza virus HA, a vector for mRNA production comprising a promoter operably linked to a DNA segment encoding influenza virus NA, a vector for mRNA production comprising a promoter operably linked to a DNA segment encoding influenza virus M1, a vector for mRNA production comprising a promoter operably linked to a DNA segment encoding influenza virus M2, or a vector for mRNA production comprising a promoter operably linked to a DNA segment encoding influenza virus NS2.

In a further embodiment, herein provided is a plurality of influenza virus vectors, comprising
a) a vector for vRNA production comprising a promoter operably linked to an influenza virus PA DNA linked to a transcription termination sequence, a vector for vRNA production comprising a promoter operably linked to an influenza virus PB1 DNA linked to a transcription termination sequence, a vector for vRNA production comprising a promoter operably linked to an influenza virus PB2 DNA linked to a transcription termination sequence, a vector for vRNA production comprising a promoter operably linked to an influenza virus HA DNA linked to a transcription termination sequence, a vector for vRNA production comprising a promoter operably linked to an influenza virus NP DNA linked to a transcription termination sequence, a vector for vRNA production comprising a promoter operably linked to an influenza virus NA DNA linked to a transcription termination sequence, a vector for vRNA production comprising a promoter operably linked to an influenza virus M DNA linked to a transcription termination sequence, and a vector for vRNA production comprising a promoter operably linked to an influenza virus NS cDNA encoding a modified NS segment, encoding an NS1 protein with a functional effector domain and a non-functional RNA binding domain with a deletion of at least 15 amino acids within the N-terminal 80 amino acids of the NS1 protein, specifically encoding any of SEQ ID NOs 3, 5, 7, 9, 11,
b) a vector for mRNA production comprising a promoter operably linked to a DNA segment encoding influenza virus PA, a vector for mRNA production comprising a promoter operably linked to a DNA segment encoding influenza virus PB1, a vector for mRNA production comprising a promoter operably linked to a DNA segment encoding influenza virus PB2, and a vector for mRNA production comprising a promoter operably linked to a DNA segment encoding influenza virus NP, and optionally a vector for mRNA production comprising a promoter operably linked to a DNA segment encoding influenza virus HA, a vector for mRNA production comprising a promoter operably linked to a DNA segment encoding influenza virus NA, a vector for mRNA production comprising a promoter operably linked to a DNA segment encoding influenza virus M1, a vector for mRNA production comprising a promoter operably linked to a DNA segment encoding influenza virus M2, or a vector for mRNA production comprising a promoter operably linked to a DNA segment encoding influenza virus NS2.

According to yet a further embodiment, herein provided is a method for preparing an influenza virus A described herein, by contacting a cell with
a) a vector for vRNA production comprising a promoter operably linked to an influenza virus PA DNA linked to a transcription termination sequence, a vector for vRNA production comprising a promoter operably linked to an influenza virus PB1 DNA linked to a transcription termination sequence, a vector for vRNA production comprising a promoter operably linked to an influenza virus PB2 DNA linked to a transcription termination sequence, a vector for vRNA production comprising a promoter operably linked to an influenza virus HA DNA linked to a transcription termination sequence, a vector for vRNA production comprising a promoter operably linked to an influenza virus NP DNA linked to a transcription termination sequence, a vector for vRNA production comprising a promoter operably linked to an influenza virus NA DNA linked to a transcription termination sequence, a vector for vRNA production comprising a promoter operably linked to an influenza virus M DNA linked to a transcription termination sequence, and a vector for vRNA production comprising a promoter operably linked to an influenza virus NS cDNA encoding a modified NS segment, encoding an NS1 protein with a functional effector domain and a non-functional RNA binding domain with a deletion of at least 15 amino acids within the N-terminal 80 amino acids of the NS1 protein, specifically encoding any of SEQ ID NOs: 3, 5, 7, 9, 11, and optionally
b) a vector for mRNA production comprising a promoter operably linked to a DNA segment encoding influenza virus PA, a vector for mRNA production comprising a promoter operably linked to a DNA segment encoding influenza virus PB1, a vector for mRNA production comprising a promoter operably linked to a DNA segment encoding influenza virus PB2, and a vector for mRNA production comprising a promoter operably linked to a DNA segment encoding influenza virus NP, and optionally a vector for mRNA production comprising a promoter operably linked to a DNA segment encoding influenza virus HA, a vector for mRNA production comprising a promoter operably linked to a DNA segment encoding influenza virus NA, a vector for mRNA production comprising a promoter operably linked to a DNA segment encoding influenza virus M1, a vector for mRNA production comprising a promoter operably linked to a DNA segment encoding influenza virus M2, or a vector for mRNA production comprising a promoter operably linked to a DNA segment encoding influenza virus NS2.

Further provided in an embodiment is a method for preparing an influenza virus A of the present invention, by contacting a cell with
a) a vector for vRNA production comprising a promoter operably linked to an influenza virus PA DNA linked to a transcription termination sequence, a vector for vRNA production comprising a promoter operably linked to an influenza virus PB1 DNA linked to a transcription termination sequence, a vector for vRNA production comprising a promoter operably linked to an influenza virus PB2 DNA linked to a transcription termination sequence, a vector for vRNA production comprising a promoter operably linked to an influenza virus HA DNA linked to a transcription termination sequence, a vector for vRNA production comprising a promoter operably linked to an influenza virus NP DNA linked to a transcription termination sequence, a vector for vRNA production comprising a promoter operably linked to an influenza virus NA DNA linked to a transcription termination sequence, a vector for vRNA production comprising a promoter operably linked to an influenza virus M DNA linked to a transcription termination sequence, and a vector for vRNA production comprising a promoter operably linked to an influenza virus NS cDNA encoding a modified NS segment, encoding an NS1 protein with a functional effector domain and a non-functional RNA binding domain with a deletion of at least 15 amino acids within the N-terminal 80 amino acids of the NS1 protein, specifically encoding any of SEQ ID NOs: 3, 5, 7, 9, 11,
b) a vector for mRNA production comprising a promoter operably linked to a DNA segment encoding influenza virus PA, a vector for mRNA production comprising a promoter operably linked to a DNA segment encoding influenza virus PB1, a vector for mRNA production comprising a promoter operably linked to a DNA segment encoding influenza virus PB2, and a vector for mRNA production comprising a promoter operably linked to a DNA segment encoding influenza virus NP, and optionally a vector for mRNA production comprising a promoter operably linked to a DNA segment encoding influenza virus HA, a vector for mRNA production comprising a promoter operably linked to a DNA segment encoding influenza virus NA, a vector for mRNA production comprising a promoter operably linked to a DNA segment encoding influenza virus M1, a vector for mRNA production comprising a promoter operably linked to a DNA segment encoding influenza virus M2, or a vector for mRNA production comprising a promoter operably linked to a DNA segment encoding influenza virus NS2.

In a further aspect, provided herein is a method for preparing an influenza virus A described herein, by contacting a cell with
a) a vector for vRNA production comprising a promoter operably linked to an influenza virus PA DNA linked to a transcription termination sequence, a vector for vRNA production comprising a promoter operably linked to an influenza virus PB1 DNA linked to a transcription termination sequence, a vector for vRNA production comprising a promoter operably linked to an influenza virus PB2 DNA linked to a transcription termination sequence, a vector for vRNA production comprising a promoter operably linked to an influenza virus HA DNA linked to a transcription termination sequence, a vector for vRNA production comprising a promoter operably linked to an influenza virus NP DNA linked to a transcription termination sequence, a vector for vRNA production comprising a promoter operably linked to an influenza virus NA DNA linked to a transcription termination sequence, a vector for vRNA production comprising a promoter operably linked to an influenza virus M DNA linked to a transcription termination sequence, and a vector for vRNA production comprising a promoter operably linked to an influenza virus NS cDNA encoding a recombinant influenza virus comprising a modified NS segment, encoding a fusion protein comprising from its N to C-terminus an NS1 protein with a functional effector domain and a non-functional RNA binding domain with a deletion of at least 15 amino acids within the N-terminal 80 amino acids of the NS1 protein a vector for mRNA production comprising a promoter operably linked to a DNA segment encoding influenza virus PA, a vector for mRNA production comprising a promoter operably linked to a DNA segment encoding influenza virus PB1 , a vector for mRNA production comprising a promoter operably linked to a DNA segment encoding influenza virus PB2, and a vector for mRNA production comprising a promoter operably linked to a DNA segment encoding influenza virus NP, and optionally a vector for mRNA production comprising a promoter operably linked to a DNA segment encoding influenza virus HA, a vector for mRNA production comprising a promoter operably linked to a DNA segment encoding influenza virus NA, a vector for mRNA production comprising a promoter operably linked to a DNA segment encoding influenza virus M1, a vector for mRNA production comprising a promoter operably linked to a DNA segment encoding influenza virus M2, or a vector for mRNA production comprising a promoter operably linked to a DNA segment encoding influenza virus NS2.

### EXAMPLES

The examples described herein are illustrative of the present invention and are not intended to be limitations thereon. Different embodiments of the present invention have been described according to the present invention.

Accordingly, it should be understood that the examples are illustrative only and are not limiting upon the scope of the invention.

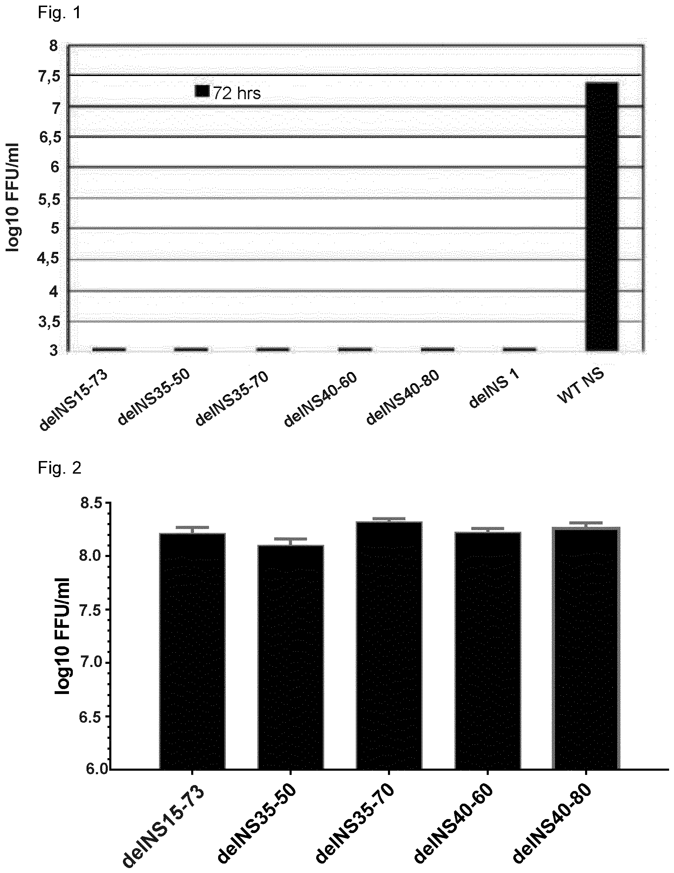

**Example 1: Growth of influenza wild-type and NS1 deletion mutants in interferon-competent human fibroblasts.** Influenza wild-type virus, an NS1 deletion mutant lacking the complete NS1 gene and different NS1 deletion mutants were analyzed for their growth properties on interferon-competent human fibroblasts after infection at an MOI of 0.01 in the presence of 0.5% 10xTrypLE in the media. In contrast to the wild-type virus that grew to a titer of 5.55 Iog10 after 24 hours post infection (p.i.), 7.52 after 48 hours p.i. and 7.61 72 hours p.i., none of the NS1 deletion mutants were detectable at any time point. The results 72 hours p.i. are shown in Figure 1 and indicate that despite having only partial NS1 deletions all the designed candidates had the desirable phenotype of being replication-deficient.

The nucleotide sequences of NS genes encoding the NS1 proteins and the amino acid sequences of the NS1 proteins of the present invention are as follows:
Amino acid sequence DeINS15-73: 
MDPNTVSSFQVDCFDEALKMTMASVPASRYLTDMTLEEMSRDWSMLIPKQKV
AGPLCIRMDQAIMDKNIILKANFSVIFDRLETLILLRAFTEEGAIVGEISPLPSLPGHTAE
DVKNAVGVLIGGLEWNDNTVRVSETLQRFAWRSSNENGRPPLTPKQKREMAGTIRS
EV (SEQ ID NO:3)
Nt sequence DeINS15-73: 
AGCAAAAGCAGGGTGACAAAGACATAATGGATCCAAACACTGTGTCAAGCT
TTCAGGTAGATTGCTTTGATGAGGCACTTAAAATGACCATGGCCTCTGTACCTGC
GTCGCGTTACCTAACTGACATGACTCTTGAGGAAATGTCAAGGGACTGGTCCATG
CTCATACCCAAGCAGAAAGTGGCAGGCCCTCTTTGTATCAGAATGGACCAGGCGA
TCATGGATAAGAACATCATACTGAAAGCGAACTTCAGTGTGATTTTTGACCGGCTG
GAGACTCTAATATTGCTAAGGGCTTTCACCGAAGAGGGAGCAATTGTTGGCGAAA
TTTCACCATTGCCTTCTCTTCCAGGACATACTGCTGAGGATGTCAAAAATGCAGTT
GGAGTCCTCATCGGGGGACTTGAATGGAATGATAACACAGTTCGAGTCTCTGAAA
CTCTACAGAGATTCGCTTGGAGAAGCAGTAATGAGAATGGGAGACCTCCACTCAC
TCCAAAACAGAAACGAGAAATGGCGGGAACAATTAGGTCAGAAGTTTGAAGAAAT
AAGATGGTTGATTGAAGAAGTGAGACACAAACTGAAGATAACAGAGAATAGTTTT
GAGCAAATAACATTTATGCAAGCCTTACATCTATTGCTTGAAGTGGAGCAAGAGAT
AAGAACTTTCTCGTTTCAGCTTATTTAATAATAAAAAACACCCTTGTTTCTACT
(SEQ ID NO:4)
Amino acid sequence of DeINS35-50 
MDPNTVSSFQVDCFLWHVRKRVADQELGDAPFLDGLDIETATRAGKQIVERIL
KEESDEALKMTMASVPASRYLTDMTLEEMSRDWSMLIPKQKVAGPLCIRMDQAIMDK
 
NIILKANFSVIFDRLETLILLRAFTEEGAIVGEISPLPSLPGHTAEDVKNAVGVLIGGLEW
NDNTVRVSETLQRFAWRSSNENGRPPLTPKQKREMAGTIRSEV (SEQ ID NO:5)
Nt sequence of DeINS35-50 
AGCAAAAGCAGGGTGACAAAGACATAATGGATCCAAACACTGTGTCAAGCT
TTCAGGTAGATTGCTTTCTTTGGCATGTCCGCAAACGAGTTGCAGACCAAGAACT
AGGTGATGCCCCATTCCTTGATGGTCTGGACATCGAGACAGCCACACGTGCTGG
AAAGCAGATAGTGGAGCGGATTCTGAAAGAAGAATCCGATGAGGCACTTAAAATG
ACCATGGCCTCTGTACCTGCGTCGCGTTACCTAACTGACATGACTCTTGAGGAAA
TGTCAAGGGACTGGTCCATGCTCATACCCAAGCAGAAAGTGGCAGGCCCTCTTTG
TATCAGAATGGACCAGGCGATCATGGATAAGAACATCATACTGAAAGCGAACTTC
AGTGTGATTTTTGACCGGCTGGAGACTCTAATATTGCTAAGGGCTTTCACCGAAG
AGGGAGCAATTGTTGGCGAAATTTCACCATTGCCTTCTCTTCCAGGACATACTGCT
GAGGATGTCAAAAATGCAGTTGGAGTCCTCATCGGGGGACTTGAATGGAATGATA
ACACAGTTCGAGTCTCTGAAACTCTACAGAGATTCGCTTGGAGAAGCAGTAATGA
GAATGGGAGACCTCCACTCACTCCAAAACAGAAACGAGAAATGGCGGGAACAATT
AGGTCAGAAGTTTGAAGAAATAAGATGGTTGATTGAAGAAGTGAGACACAAACTG
AAGATAACAGAGAATAGTTTTGAGCAAATAACATTTATGCAAGCCTTACATCTATTG
CTTGAAGTGGAGCAAGAGATAAGAACTTTCTCGTTTCAGCTTATTTAATAATAAAAA
ACACCCTTGTTTCTACT (SEQ ID NO:6)
Amino acid sequence of DeINS35-70 
MDPNTVSSFQVDCFLWHVRKRVADQELGDAPFLDEESDEALKMTMASVPAS
RYLTDMTLEEMSRDWSMLIPKQKVAGPLCIRMDQAIMDKNIILKANFSVIFDRLETLILL
RAFTEEGAIVGEISPLPSLPGHTAEDVKNAVGVLIGGLEWNDNTVRVSETLQRFAWR
SSNENGRPPLTPKQKREMAGTIRSEV (SEQ ID NO:7)
Nt sequence of DeINS35-70 
AGCAAAAGCAGGGTGACAAAGACATAATGGATCCAAACACTGTGTCAAGCT
TTCAGGTAGATTGCTTTCTTTGGCATGTCCGCAAACGAGTTGCAGACCAAGAACT
AGGTGATGCCCCATTCCTTGATGAAGAATCCGATGAGGCACTTAAAATGACCATG
GCCTCTGTACCTGCGTCGCGTTACCTAACTGACATGACTCTTGAGGAAATGTCAA
GGGACTGGTCCATGCTCATACCCAAGCAGAAAGTGGCAGGCCCTCTTTGTATCAG
AATGGACCAGGCGATCATGGATAAGAACATCATACTGAAAGCGAACTTCAGTGTG
 
ATTTTTGACCGGCTGGAGACTCTAATATTGCTAAGGGCTTTCACCGAAGAGGGAG
CAATTGTTGGCGAAATTTCACCATTGCCTTCTCTTCCAGGACATACTGCTGAGGAT
GTCAAAAATGCAGTTGGAGTCCTCATCGGGGGACTTGAATGGAATGATAACACAG
TTCGAGTCTCTGAAACTCTACAGAGATTCGCTTGGAGAAGCAGTAATGAGAATGG
GAGACCTCCACTCACTCCAAAACAGAAACGAGAAATGGCGGGAACAATTAGGTCA
GAAGTTTGAAGAAATAAGATGGTTGATTGAAGAAGTGAGACACAAACTGAAGATA
ACAGAGAATAGTTTTGAGCAAATAACATTTATGCAAGCCTTACATCTATTGCTTGAA
GTGGAGCAAGAGATAAGAACTTTCTCGTTTCAGCTTATTTAATAATAAAAAACACC
CTTGTTTCTACT (SEQ ID NO:8)
Amino acid sequence of DeINS40-60 
MDPNTVSSFQVDCFLWHVRKRVADQELGDAPFLDRLRRDGKQIVERILKEES
DEALKMTMASVPASRYLTDMTLEEMSRDWSMLIPKQKVAGPLCIRMDQAIMDKNIILK
ANFSVIFDRLETLILLRAFTEEGAIVGEISPLPSLPGHTAEDVKNAVGVLIGGLEWNDNT
VRVSETLQRFAWRSSNENGRPPLTPKQKREMAGTIRSEV (SEQ ID NO:9)
Nt sequence of DeINS40-60 
AGCAAAAGCAGGGTGACAAAGACATAATGGATCCAAACACTGTGTCAAGCT
TTCAGGTAGATTGCTTTCTTTGGCATGTCCGCAAACGAGTTGCAGACCAAGAACT
AGGTGATGCCCCATTCCTTGATCGGCTTCGCCGAGATGGAAAGCAGATAGTGGA
GCGGATTCTGAAAGAAGAATCCGATGAGGCACTTAAAATGACCATGGCCTCTGTA
CCTGCGTCGCGTTACCTAACTGACATGACTCTTGAGGAAATGTCAAGGGACTGGT
CCATGCTCATACCCAAGCAGAAAGTGGCAGGCCCTCTTTGTATCAGAATGGACCA
GGCGATCATGGATAAGAACATCATACTGAAAGCGAACTTCAGTGTGATTTTTGACC
GGCTGGAGACTCTAATATTGCTAAGGGCTTTCACCGAAGAGGGAGCAATTGTTGG
CGAAATTTCACCATTGCCTTCTCTTCCAGGACATACTGCTGAGGATGTCAAAAATG
CAGTTGGAGTCCTCATCGGGGGACTTGAATGGAATGATAACACAGTTCGAGTCTC
TGAAACTCTACAGAGATTCGCTTGGAGAAGCAGTAATGAGAATGGGAGACCTCCA
CTCACTCCAAAACAGAAACGAGAAATGGCGGGAACAATTAGGTCAGAAGTTTGAA
GAAATAAGATGGTTGATTGAAGAAGTGAGACACAAACTGAAGATAACAGAGAATA
GTTTTGAGCAAATAACATTTATGCAAGCCTTACATCTATTGCTTGAAGTGGAGCAA
GAGATAAGAACTTTCTCGTTTCAGCTTATTTAATAATAAAAAACACCCTTGTTTCTA
CT (SEQ ID NO:10)
Amino acid sequence of DeINS40-80 
MDPNTVSSFQVDCFLWHVRKRVADQELGDAPFLDRLRRDMASVPASRYLTD
MTLEEMSRDWSMLIPKQKVAGPLCIRMDQAIMDKNIILKANFSVIFDRLETLILLRAFTE
EGAIVGEISPLPSLPGHTAEDVKNAVGVLIGGLEWNDNTVRVSETLQRFAWRSSNEN
GRPPLTPKQKREMAGTIRSEV (SEQ ID NO:11)
Nt sequence of DeINS40-80

AGCAAAAGCAGGGTGACAAAGACATAATGGATCCAAACACTGTGTCAAGCT
TTCAGGTAGATTGCTTTCTTTGGCATGTCCGCAAACGAGTTGCAGACCAAGAACT
AGGTGATGCCCCATTCCTTGATCGGCTTCGCCGAGATATGGCCTCTGTACCTGCG
TCGCGTTACCTAACTGACATGACTCTTGAGGAAATGTCAAGGGACTGGTCCATGC
TCATACCCAAGCAGAAAGTGGCAGGCCCTCTTTGTATCAGAATGGACCAGGCGAT
CATGGATAAGAACATCATACTGAAAGCGAACTTCAGTGTGATTTTTGACCGGCTG
GAGACTCTAATATTGCTAAGGGCTTTCACCGAAGAGGGAGCAATTGTTGGCGAAA
TTTCACCATTGCCTTCTCTTCCAGGACATACTGCTGAGGATGTCAAAAATGCAGTT
GGAGTCCTCATCGGGGGACTTGAATGGAATGATAACACAGTTCGAGTCTCTGAAA
CTCTACAGAGATTCGCTTGGAGAAGCAGTAATGAGAATGGGAGACCTCCACTCAC
TCCAAAACAGAAACGAGAAATGGCGGGAACAATTAGGTCAGAAGTTTGAAGAAAT
AAGATGGTTGATTGAAGAAGTGAGACACAAACTGAAGATAACAGAGAATAGTTTT
GAGCAAATAACATTTATGCAAGCCTTACATCTATTGCTTGAAGTGGAGCAAGAGAT
AAGAACTTTCTCGTTTCAGCTTATTTAATAATAAAAAACACCCTTGTTTCTACT
(SEQ ID NO:12)

Figure 1 shows the growth of NS1 deletion mutants in interferon-competent cells. Human fibroblasts were infected with influenza wild-type virus (WT NS), and different deletion mutants: deINS1 has a complete NS1 deletion, for the other deletion mutants the deleted amino acids of the NS1 protein are indicated.

**Example 2: Growth of influenza wild-type and NS1 deletion mutants in interferon-deficient Vero cells**. Different NS1 deletion mutants (length of the deletion is indicated) were analyzed for their growth properties on Vero cells at an MOI of 0.001 in the presence of 0.5% 10xTrypLE in the media. Supernatants were harvested 48 hours p.i. and viral titers of the different deletion mutants were determined. Importantly, all NS1 deletion mutants grow to high titers above 8log10 per ml (Fig.2) indicating to have sufficient growth to be used in a therapeutic approach.

Figure 2 shows the growth of NS1 deletion mutants in interferon-deficient Vero cells. Cell were infected with different deletion mutants and viral titers were determined. The length of the NS1 deletion is indicated.

### Example 3:

Interferon-beta induction of NS1 deletion mutants. Influenza wild-type virus, an NS1 deletion mutant lacking the complete NS1 gene and different NS1 deletion mutants were analyzed for their ability to induce interferon (IFN)-beta upon infection of human fibroblasts after infection at an MOI of 10. Two hours post infection (p.i.) the media was supplemented with 10% FCS. 24 hours p.i. the supernatants were analyzed for the presence of IFN by ELISA. All deletion mutants induced significantly higher IFN levels than the virus lacking the complete NS1 and the wild-type virus. (Fig.3).

Figure 3 shows the interferon-beta induction by NS1 deletion mutants. Human fibroblasts were infected with wild-type influenza virus (WT NS), and different deletion mutants. deINS1 has a complete NS1 deletion, for the other deletion mutants the deleted amino acids of the NS1 protein are indicated. High amounts of > 300pg/ml IFN-beta were found for the intermediate deletion mutants but not for the complete deletion mutant or the wild-type.

### Example 4:

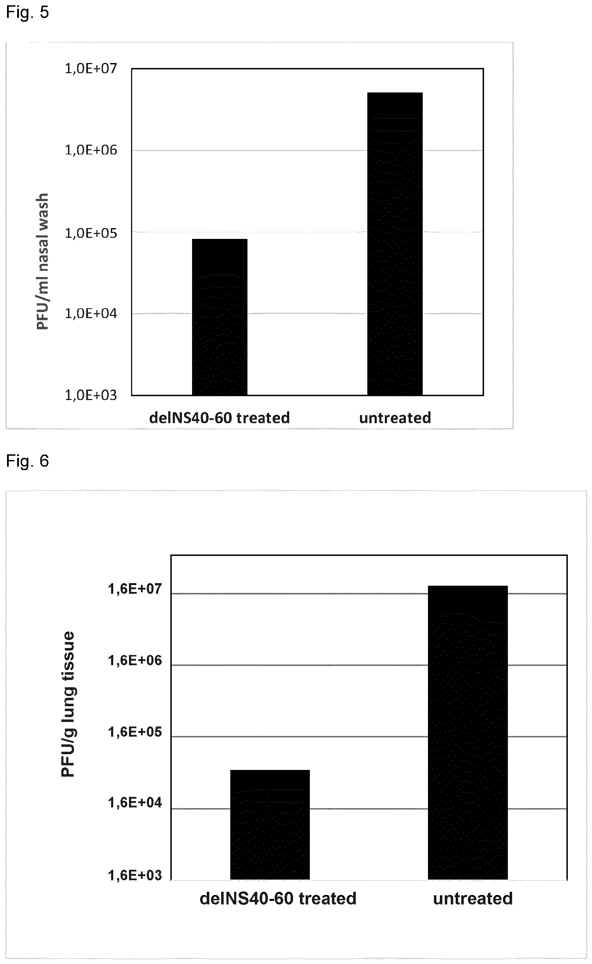

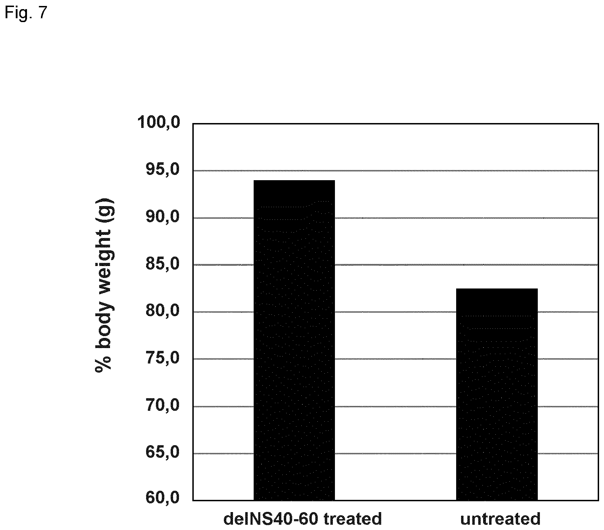

The anti-viral activity of the deINS40-60 deletion mutant (influenza A/New Caledonia virus lacking amino acids 40 to 60 of the NS1 protein) was tested in animal models. Influenza virus inhibition was tested in a ferret model and Sars-CoV-2 inhibition was tested in the mouse ACE2 model. Ferrets were treated with deINS40-60 at different time points before and after infection with pandemic influenza H1N1pdm09 wild-type virus. This treatment was protective as indicated by significant reduction of the H1 N1 pdm09 challenge virus in the nasal wash titers after challenge (Fig 5). In the mouse model, mice were treated with deINS40-60, at different time points before and/or after infection with Sars-CoV-2 virus. The treatment was protective as indicated by significant reduction of the Sars-CoV-2 challenge virus in the lungs after infection (Fig 6). Another indicator of disease, weight loss was also substantially reduced from approximately 20% to 5% by pretreatment with deINS40-60 (Fig.7).

### REFERENCES

Ali et al., 2000, Influenza virus assembly: effect of influenza virus glycoproteins on the membrane association of M1 protein. J. Virol. 74, 8709-8719. doi: 10.1128/ JVI.74.18.8709-8719.2000
Barman et al., 2004, Role of transmembrane domain and cytoplasmic tail amino acid sequences of influenza a virus neuraminidase in raft association and virus budding. J. Virol. 78, 5258-5269. doi: 10.1128ZJVI.78.10.5258-5269.2004
Cinatl J, Morgenstern B, Bauer G, Chandra P, Rabenau H, Doerr HW. Treatment of SARS with human interferons. Lancet. 2003. 362:293-4
Ekiert DC, et al., A highly conserved neutralizing epitope on group 2 influenza A viruses. Science. 2011; 333:843-850
Enami and Enami, 1996, Influenza virus hemagglutinin and neuraminidase glycoproteins stimulate the membrane association of the matrix protein. J. Virol. 70, 6653-6657
Friedman, R. M. 2008. Clinical uses of interferons. Br. J. Clin. Pharmacol. 65:158-162.
Garcia-Sastre and Palese, 1995, The cytoplasmic tail of the neuraminidase protein of influenza A virus does not play an important role in the packaging of this protein into viral envelopes. Virus Res. 37, 37-47. doi: 10.1016/0168-1702(95)00017-K
Haagmans BL, Kuiken T, Martina BE, Fouchier RA, Rimmelzwaan GF, van Amerongen G, van Riel D, de Jong T, Itamura S, Chan KH, Tashiro M, Osterhaus AD. Pegylated interferon-alpha protects type 1 pneumocytes against SARS coronavirus infection in macaques. Nat Med. 2004. 10:290-3
Jin et al., 1997; Influenza virus hemagglutinin and neuraminidase cytoplasmic tails control particle shape. EMBO J. 16, 1236-1247. doi: 10.1093/emboj/16.6.1236
Kida H. et al., Inhibition of virus-induced hemolysis with monoclonal antibodies to different antigenic areas on the hemagglutinin molecule of A/seal/Massachusetts/1/80 (H7N7) influenza virus, Arch Virol., 1983, 76(2), 91-9
Kilbourne ED, Comparative efficacy of neuraminidase-specific and conventional influenza virus vaccines in induction of antibody to neuraminidase in humans, J Infect Dis. 1976, 134(4), 384-94
Kirkpatrick E. et al., 2018, The influenza virus hemagglutinin head evolves faster than the stalk domain, Scientific Reports, 8:10432, 1-14
Krystal M., et al., 1982, of structural features in the hemagglutinin genes, Proc Natl Acad Sci USA. 1982; 79:4800-4804
Loutfy MR, Blatt LM, Siminovitch KA, Ward S, Wolff B, et. al. Interferon alfacon-1 plus corticosteroids in severe acute respiratory syndrome: a preliminary study. JAMA. 2003. 290:3222-8
McAuley J.L. et al., 2019, Influenza Virus Neuraminidase Structure and Functions, Front Microbiol., 10, 39
McKinlay, M. A. 2001. Recent advances in the treatment of rhinovirus infections. Curr. Opin. Pharmacol. 1:477-481.
Melian EB, Plosker GL. Interferon alfacon-1. Drugs 2001. 61:1661-1691.
Mintaev et al., 2014, Co-evolution analysis to predict protein-protein interactions within influenza virus envelope. J. Bioinforma. Comput. Biol. 12:1441008. doi: 10.1142/S021972001441008X
Mitnaul et al., 1996; The cytoplasmic tail of influenza A virus neuraminidase (NA) affects NA incorporation into virions, virion morphology, and virulence in mice but is not essential for virus replication. J. Virol. 70, 873-879.
Moules et al., 2010, In vitro characterization of naturally occurring influenza H3NA-viruses lacking the NA gene segment: toward a new mechanism of viral resistance? Virology 404, 215-224. doi: 10.1016/j.virol.2010.04.030
Shim J.M. et al., Influenza Virus Infection, Interferon Response, Viral Counter-Response, and Apoptosis, Viruses, 2017, 9(8), 223
Turner RB, Felton A, Kosak K, Kelsey DK, Meschievitz CK. Prevention of experimental coronavirus colds with intranasal alpha-2b interferon. J Infect Dis. 1986. 154:443-7.
Tyrell DA: The efficacy and tolerance of intranasal interferons: studies at the Common Cold Unit. J. Antimicrob. Chemother. 1986. 18:153-156.
Varghese et al., 1983; Structure of the influenza virus glycoprotein antigen neuraminidase at 2.9 A resolution. Nature 303, 35-40. doi: 10.1038/303035a0
Wang T.T. et al., Broadly protective monoclonal antibodies against H3 influenza viruses following sequential immunization with different hemagglutinins, PLoS Pathog. 2010; 6:e1000796
Ward et al., 1983; The disulphide bonds of an Asian influenza virus neuraminidase. FEBS Lett. 153, 29-33. doi: 10.1016/0014-5793(83)80113-6
Yasugi M. eet al., Emerging antigenic variants at the antigenic site Sb in pandemic A(H1N1)2009 influenza virus in Japan detected by a human monoclonal antibody, PLoS One, 2013, 16:8(10), e77892
Zhao Z, Zhang F, Xu M et al.: Description and clinical treatment of an early outbreak of severe acute respiratory syndrome (SARS) in Guangzhou, PR China. J. Med. Microbiol. 2003. 52:715-720.

## Claims

1. Replication deficient influenza A virus which is inducing high levels of type I interferon (IFN) in cells infected by said virus, comprising an NS1 protein with a functional effector domain and a non-functional RNA binding domain, with a deletion of at least 15 amino acids within the N-terminal 80 amino acids of the NS1 protein, and wherein the virus comprises the sequence of any one of SEQ ID NOs: 3, 5, 7, 9, and 11.

2. The influenza A virus according to claim 1, comprising deletions of the N-terminal amino acids 15 to 73, 35 to 50, 35 to 70, 40 to 60, or 40 to 80 with reference to the numbering of SEQ ID NO:1.

3. The influenza A virus according to any one of claims 1 to 2, further comprising modifications of the NA, HA, PB1, PB2, PA and/or NP proteins.

4. Pharmaceutical preparation comprising the influenza A virus according to any one of claims 1 to 3, optionally in combination with a pharmaceutically acceptable carrier.

5. The influenza A virus according to any one of claims 1 to 3, or the pharmaceutical preparation according to claim 4, for use in the preparation of a medicament.

6. The influenza A virus or pharmaceutical preparation for use according to claim 5 in antiviral treatment of a subject, specifically in the prophylactic or therapeutic treatment of an infection caused by IFN-sensitive viruses.

7. The influenza virus or the pharmaceutical preparation for use according to claim 6, wherein said IFN-sensitive viruses are selected from the group consisting of coronavirus, influenza virus, respiratory syncytial virus, metapneumovirus, parainfluenza virus, flaviviruses, hepatitis virus, herpes simplex virus, rhinovirus and vaccinia virus.

8. The influenza virus or the pharmaceutical preparation for use according to claim 6 or 7, wherein the coronavirus is a β-coronavirus, SARS-CoV-2, MERS-CoV, SARS-CoV-1, HCoV-OC43, HCoV-HKU1, or an α-coronavirus, HCoV-NL63, HCoV-229E, or PEDV.

9. The influenza virus according to any one of claims 1 to 3 for use in the treatment of a disease condition caused by or associated with SARS-CoV-2.

10. The influenza virus or the pharmaceutical preparation for use according to any one of claims 6 to 9 in the treatment of a disease condition caused by an IFN-sensitive virus, wherein said disease condition is influenza, common cold, infection of the nose, sinusitis, throat and larynx, bronchiolitis, diarrhea, rash on skin, or pneumonia, acute respiratory distress syndrome (ARDS).

11. The pharmaceutical preparation according to claim 4, which is formulated for local administration, preferably for application to the upper and lower respiratory tract, nasal, pulmonary, intraoral, ocular, or dermal use, or for systemic administration, preferably for intravenous, intramuscular, subcutaneous, intradermal, transdermal, or oral administration, specifically it is formulated for nasal administration.

12. The pharmaceutical preparation according to claim 11, wherein said pharmaceutical preparation is formulated as a spray, a powder, a gel, an ointment, a cream, a foam, or a liquid solution, a lotion, a gargle solution, an aerosolized powder, an aerosolized liquid formulation, granules, capsules, drops, tablet, syrup, lozenge, or a preparation for infusion or injection.

13. The pharmaceutical preparation according to claim 4, wherein said preparation is for administration as the sole antiviral substance, or wherein preparation is combined with a further preparation comprising one or more active substances, preferably selected from the group consisting of antiviral, and antibiotic substances.

14. The pharmaceutical preparation for use according to claim 6, wherein the subject has been infected or is at risk of being infected with an IFN-sensitive virus, preferably it is a human being, dog, cat, horse, camelid, cattle or pig.

15. The influenza A virus according to any one of claims 1 to 3, or the pharmaceutical preparation according to claim 4 for use in inhibiting replication of IFN sensitive viruses.

16. An isolated nucleic acid sequence expressing the replication deficient influenza A virus according to any one of claims 1 to 3.

17. The isolated nucleic acid sequence according to claim 16, comprising any one of SEQ ID NOs: 4, 6, 8, 10, and 12.

## Patentansprüche

1. Replikationsdefizientes Influenza-A-Virus, das hohe Niveaus von Interferon (IFN) Typ I in Zellen induziert, die mit dem Virus infiziert sind, umfassend ein NS1-Protein mit einer funktionellen Effektordomäne und einer nicht funktionellen RNA-Bindungsdomäne, mit einer Deletion von mindestens 15 Aminosäuren innerhalb der 80 N-terminalen Aminosäuren des NS1-Proteins, und wobei das Virus die Sequenz nach einer der SEQ ID NO: 3, 5, 7, 9 und 11 umfasst.

2. Influenza-A-Virus nach Anspruch 1, umfassend Deletionen der N-terminalen Aminosäuren 15 bis 73, 35 bis 50, 35 bis 70, 40 bis 60 oder 40 bis 80 bezogen auf die Nummerierung von SEQ ID NO:1.

3. Influenza-A-Virus nach einem der Ansprüche 1 bis 2, ferner umfassend Modifikationen der NA-, HA-, PB1-, PB2-, PA- und/oder NP-Proteine.

4. Pharmazeutische Zubereitung, umfassend das Influenza-A-Virus nach einem der Ansprüche 1 bis 3, gegebenenfalls in Kombination mit einem pharmazeutisch annehmbaren Träger.

5. Influenza-A-Virus nach einem der Ansprüche 1 bis 3, oder die pharmazeutische Zubereitung nach Anspruch 4, zur Verwendung bei der Herstellung eines Medikaments.

6. Influenza-A-Virus oder pharmazeutische Zubereitung zur Verwendung nach Anspruch 5 bei der antiviralen Behandlung eines Subjekts, im Speziellen bei der prophylaktischen oder therapeutischen Behandlung einer durch IFN-sensitive Viren verursachten Infektion.

7. Influenza-A-Virus oder pharmazeutische Zubereitung zur Verwendung nach Anspruch 6, wobei die IFN-sensitiven Viren aus der Gruppe bestehend aus Corona-Virus, Influenza-Virus, respiratorischen Synzytial-Virus, Metapneumo-Virus, Parainfluenza-Virus, Flavi-Viren, Hepatitis-Virus, Herpes-simplex-Virus, Rhino-Virus und Vaccinia-Virus ausgewählt sind.

8. Influenza-Virus oder pharmazeutische Zubereitung zur Verwendung nach Anspruch 6 oder 7, wobei das Corona-Virus ein β-Corona-Virus, SARS-CoV-2, MERS-CoV, SARS-CoV-1, HCoV-OC43, HCoV-HKU1, oder ein α-Corona-Virus, HCoV-NL63, HCOV-229E oder PEDV ist.

9. Influenza-Virus nach einem der Ansprüche 1 bis 3 zur Verwendung bei der Behandlung eines Krankheitszustands, der von SARS-CoV-2 verursacht wird oder damit assoziiert ist.

10. Influenza-Virus oder pharmazeutische Zubereitung zur Verwendung nach einem der Ansprüche 6 bis 9 bei der Behandlung eines von einem IFN-sensitiven Virus verursachten Krankheitszustands, wobei der Krankheitszustand Influenza, gewöhnliche Erkältung, Infektion der Nase, Sinusitis, des Rachens und Kehlkopfs, Bronchiolitis, Diarrhöe, Ausschlag auf der Haut, oder Pneumonie, akutes Atemnotsyndrom (ARDS) ist.

11. Pharmazeutische Zubereitung nach Anspruch 4, die für die lokale Verabreichung formuliert ist, vorzugsweise zur Aufbringung auf die oberen und unteren Atemwege, die nasale, pulmonale, intraorale, okulare oder dermale Verwendung, oder für die systemische Verabreichung, vorzugsweise durch intravenöse, intramuskuläre, subkutane, intradermale, transdermale oder orale Verabreichung, wobei sie im Speziellen für die nasale Verabreichung formuliert ist.

12. Pharmazeutische Zubereitung nach Anspruch 11, wobei die pharmazeutische Zubereitung als ein Spray, ein Pulver, ein Gel, eine Salbe, eine Creme, ein Schaum oder eine flüssige Lösung, eine Lotion, eine Gurgellösung, ein zerstäubtes Pulver, eine zerstäubte flüssige Formulierung, als Granulat, Kapseln, Tropfen, Tablette, Sirup, Lutschtablette oder als Zubereitung für die Infusion oder Injektion formuliert ist.

13. Pharmazeutische Zubereitung nach Anspruch 4, wobei die Zubereitung für die Verabreichung als einzige antivirale Substanz vorgesehen ist, oder wobei die Zubereitung mit einer weiteren Zubereitung kombiniert wird, die eine oder mehrere aktive Substanzen umfasst, die vorzugsweise aus der Gruppe bestehend aus antiviralen und antibiotischen Substanzen ausgewählt sind.

14. Pharmazeutische Zubereitung zur Verwendung nach Anspruch 6, wobei das Subjekt mit einem IFN-sensitiven Virus infiziert ist oder das Risiko besteht, dass es damit infiziert wird, wobei es vorzugsweise ein menschliches Wesen, ein Hund, eine Katze, ein Pferd, ein Kamelid, ein Rind oder Schwein ist.

15. Influenza-A-Virus nach einem der Ansprüche 1 bis 3, oder pharmazeutische Zubereitung nach Anspruch 4, zur Verwendung bei der Hemmung der Replikation von IFN-sensitiven Viren.

16. Isolierte Nukleinsäuresequenz, welche das replikationsdefiziente Influenza-A-Virus nach einem der Ansprüche 1 bis 3 exprimiert.

17. Isolierte Nukleinsäuresequenz nach Anspruch 16, umfassend eine beliebige der SEQ ID NO: 4, 6, 8, 10 und 12.

## Revendications

1. Virus de la grippe A à réplication déficiente qui induit des taux élevés d'interféron (IFN) de type I dans les cellules infectées par ledit virus, comprenant une protéine NS1 avec un domaine effecteur fonctionnel et un domaine de liaison à l'ARN non fonctionnel, avec une délétion d'au moins 15 acides aminés dans les 80 acides aminés N-terminaux de la protéine NS1, et dans lequel le virus comprend la séquence selon l'une quelconque des SEQ ID N° : 3, 5, 7, 9 et 11.

2. Virus de la grippe A selon la revendication 1, comprenant des délétions des acides aminés N-terminaux 15 à 73, 35 à 50, 35 à 70, 40 à 60, ou 40 à 80 en référence à la numérotation de SEQ ID N° : 1.

3. Virus de la grippe A selon l'une quelconque des revendications 1 à 2, comprenant en outre des modifications des protéines NA, HA, PB1, PB2, PA et/ou NP.

4. Préparation pharmaceutique comprenant le virus de la grippe A selon l'une quelconque des revendications 1 à 3, éventuellement en combinaison avec un vecteur pharmaceutiquement acceptable.

5. Virus de la grippe A selon l'une quelconque des revendications 1 à 3, ou préparation pharmaceutique selon la revendication 4, destiné à être utilisé dans la préparation d'un médicament.

6. Virus de la grippe A ou préparation pharmaceutique destiné à être utilisé selon la revendication 5 dans le traitement antiviral d'un sujet, en particulier dans le traitement prophylactique ou thérapeutique d'une infection provoquée par les virus sensibles à l'IFN.

7. Virus de la grippe ou préparation pharmaceutique destiné à être utilisé selon la revendication 6, dans laquelle lesdits virus sensibles à l'IFN sont choisis dans le groupe constitué par un coronavirus, le virus de la grippe, le virus respiratoire syncytial, le métapneumovirus, le virus paragrippal, les flavivirus, le virus de l'hépatite, le virus de l'herpès simplex, un rhinovirus et le virus de la vaccine.

8. Virus de la grippe ou préparation pharmaceutique destiné à être utilisé selon la revendication 6 ou 7, dans laquelle le coronavirus est un β-coronavirus, le SARS-CoV-2, le MERS-CoV, le SARS-CoV-1, le HCoV-OC43, le HCoV-HKU1, ou un α-coronavirus, le HCoV-NL63, le HCoV-229E ou le VDEP.

9. Virus de la grippe selon l'une quelconque des revendications 1 à 3 destiné à être utilisé dans le traitement d'un état pathologique causé ou associé au SARAS-CoV-2.

10. Virus de la grippe ou préparation pharmaceutique destiné à être utilisé selon l'une quelconque des revendications 6 à 9 dans le traitement d'un état pathologique causé par un virus sensible à l'IFN, dans laquelle ledit état pathologique est la grippe, un rhume simple, une infection du nez, une sinusite, la gorge et le larynx, une bronchiolite, une diarrhée, une éruption cutanée, ou une pneumonie, le syndrome de détresse respiratoire aiguë (SDRA).

11. Préparation pharmaceutique selon la revendication 4, qui est formulée pour une administration locale, de préférence pour une application dans les voies aériennes supérieures et inférieures, une utilisation nasale, pulmonaire, intrabuccale, oculaire ou dermique, ou pour une administration systémique, de préférence pour une administration intraveineuse, intramusculaire, sous-cutanée, intradermique, transdermique ou orale, en particulier celle-ci est formulée pour une administration nasale.

12. Préparation pharmaceutique selon la revendication 11, dans laquelle ladite préparation pharmaceutique est formulée sous la forme d'un spray, d'une poudre, d'un gel, d'une pommade, d'une crème, d'une mousse ou d'une solution liquide, d'une lotion, d'une solution de gargarisme, d'une poudre aérosolisée, d'une formulation liquide aérosolisée, de granulés, de capsules, de gouttes, d'un comprimé, d'un sirop, d'une pastille ou d'une préparation pour perfusion ou injection.

13. Préparation pharmaceutique selon la revendication 4, dans laquelle ladite préparation pharmaceutique est destinée à une administration en tant que seule substance antivirale, ou dans laquelle la préparation est combinée à une préparation supplémentaire comprenant une ou plusieurs substances actives, de préférence choisies dans le groupe constitué par les substances antivirales et antibiotiques.

14. Préparation pharmaceutique destinée à être utilisée selon la revendication 6, dans laquelle le sujet a été infecté ou présente un risque d'être infecté par un virus sensible à l'IFN, de préférence celui-ci est un être humain, un chien, un chat, un cheval, un camélidé, un bovin ou un porc.

15. Virus de la grippe A selon l'une quelconque des revendications 1 à 3, ou préparation pharmaceutique selon la revendication 4, destiné à être utilisé dans l'inhibition de la réplication des virus sensibles à l'IFN.

16. Séquence d'acide nucléique isolée exprimant le virus de la grippe A à réplication déficiente selon l'une quelconque des revendications 1 à 3.

17. Séquence d'acide nucléique isolée selon la revendication 16, comprenant l'une quelconque des SEQ ID N° : 4, 6, 8, 10 et 12.
